# EUROPEAN PATENT SPECIFICATION

(11) **EP 4 484 454 B1**
(45) Date of publication and mention of the grant of the patent: **11.02.2026**
(21) Application number: 23765930.5
(22) Date of filing: 06.03.2023
(51) Int. Cl.: C08F 2/48, C08F 20/10, C09D 4/00, G03F 7/028, C07C 225/22

(54) **BENZOPHENONE DERIVATIVE, METHOD FOR PREPARING SAME, AND USE THEREOF**
BENZOPHENONDERIVAT, VERFAHREN ZU SEINER HERSTELLUNG UND SEINE VERWENDUNG
DÉRIVÉ DE BENZOPHÉNONE, SON PROCÉDÉ DE PRÉPARATION ET SON UTILISATION

(30) Priority: 07.03.2022 CN 202210217084
(43) Date of publication of application: 01.01.2025
(73) Proprietor: IGM (ANQING) HIGH TECHNOLOGY DEVELOPMENT CO., LTD., Anqing, Anhui 246002 (CN); Insight High Technology (Beijing) Co., Ltd., Haidian District Beijing 100095 (CN)
(72) Inventor: MA, Zhongli, Anqing, Anhui 246002 (CN); ZHAO, Wenchao, Anqing, Anhui 246002 (CN); WANG, Yonglin, Anqing, Anhui 246002 (CN); LYU, Dongxu, Anqing, Anhui 246002 (CN); SHAO, Junfeng, Anqing, Anhui 246002 (CN); WANG, Chenlong, Anqing, Anhui 246002 (CN); HU, Weijing, Anqing, Anhui 246002 (CN); LI, Jiaqi, Anqing, Anhui 246002 (CN)
(74) Representative: Petty, Catrin Helen
(86) International application number: PCT/CN2023/079796
(87) International publication number: WO 2023/169352

(56) References cited:
- CN-A- 1 844 087
- CN-A- 101 029 095
- CN-A- 102 212 151
- CN-A- 105 319 848
- CN-A- 112 940 242
- GB-A- 2 263 909
- US-A- 4 507 497
- WANG, HONGYU ET AL.: "Novel Polymerizable N-aromatic Maleimides as Free Radical Initiators for Phtopolymerization", POLYMER INTERNATIONAL, vol. 55, no. 8, 23 May 2006 (2006-05-23), XP055506278, ISSN: 0959-8103, DOI: 10.1002/pi.2041

## Description

### CROSS REFERENCE TO RELATED APPLICATIONS

The present application claims priority to Chinese Patent Application No. 202210217084.9, entitled "BENZOPHENONE DERIVATIVE, METHOD FOR PREPARING SAME, AND USE THEREOF", and filed to the China National Intellectual Property Administration on March 7, 2022.

### TECHNICAL FIELD

The present application relates to the technical field of photocuring technique, in particular to a benzophenone derivative, a method for preparing same, and use thereof.

### BACKGROUND

N,N,N,N-tetraethyl-4,4'-diaminobenzophenone, referred to as EMK, is a commonly used high-efficiency auxiliary photoinitiator, which is very important in inks, especially UV-LED curing inks. The synthesis methods of the compounds are reported in patent documents such as CN107686450A, CN112707830A, DE2226039A1 and DE44077C. In patents EP1078598A1, US2010081071A1, CN105974736A, CN104749882A, CN104710843A, EMK is used as an auxiliary photoinitiator and in combination with a hydrogen abstraction photoinitiator in various combinations to achieve photopolymerization. However, its disadvantages are that it has a small molecular weight and a certain degree of toxicity, and it is easy to migrate out of the cured material, affecting the stability and safety of the product properties.

In patent CN101796015A, an acrylate-based derivatized aniline compound is reported as a polymerizable ammonia additive for use in radiation-curable liquid compositions for inkjet printing. Its disadvantages are small molecular weight, complex preparation process and high manufacturing cost. In patent CN102212151A, a 4-acrylamido-4'-dialkylaminobenzophenone compound is reported. It is reported that it can be used alone as a photoinitiator. The disadvantage is that the photoinitiation efficiency is not high and the preparation process is complicated.

In the actual use of photocurable inks, after people noticed the pollution problems of small molecular weight photoinitiators, the demand for low volatility and low mobility photoinitiators continued to increase with the expansion of the amount of coatings, such as requirements for low odor and low mobility inks are widely used in civilian fields such as paper and flooring, especially for food and drug packaging materials, more stringent testing standards limit the amount of substance that can migrate. Therefore, it is difficult to meet the strict mobility standard requirements and is not included in the list of allowed uses. It also makes many users lose efficient formula combinations. It is difficult to find alternative technologies that meet the standards for a while, which has become a problem for those skilled in the art.

### SUMMARY OF THE INVENTION

The technical problem to be solved by the present application is to overcome the defect of high mobility amount of photoinitiator or auxiliary photoinitiator in the prior art, thereby providing a benzophenone derivative, a method for preparing same, and use thereof.

In order to solve the above technical problems, the present application adopts the following technical solutions:
A benzophenone derivative, wherein the benzophenone derivative has a structure represented by formula (1): 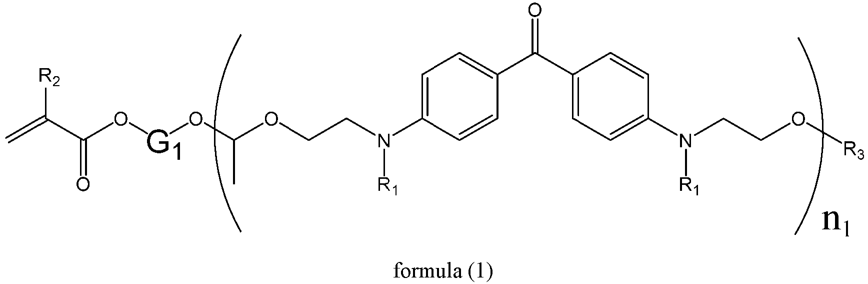 wherein:
n₁ is an integer from 1 to 10, R₁ is an optionally substituted C1-C8 alkyl, R₂ is H or -CH₃,
R₃ is H or 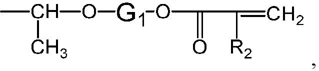 G₁ is an optionally substituted C1-C12 alkylene, 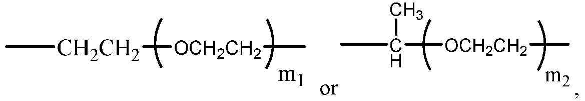 m₁ is an integer from 1 to 11, and m₂ is an integer from 1 to 12.

The term "substituted" means that any one or more hydrogen atoms on a specified atom are replaced by a substituent, as long as the substituted compound is stable. The term "optionally substituted" means that it may or may not be substituted. Unless otherwise specified, the type and number of substituents may be arbitrary on the basis that they can be chemically realized.

In the present application, the alkyl may be a linear alkyl or a branched alkyl.

In the present application, n₁ is an integer from 1 to 10, such as 1, 2, 3, 4, 5, 6, 7, 8, 9 or 10. In an optional embodiment of the present application, n₁ is an integer from 1 to 4.

In R₁, examples of C1-C8 alkyls include but are not limited to methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2,2-dimethyl- 1-butyl, 3,3-dimethyl-1-butyl, 2-ethyl-1-butyl, n-butyl, isobutyl, sec-butyl, tert-butyl, n-pentyl, isopentyl, neopentyl, etc. In some embodiments, alkyl is C1-C8 alkyl, C1-C7 alkyl, C1-C6 alkyl, C1-C5 alkyl, C1-C4 alkyl, C1-C3 alkyl, C1-C2 alkyl or C1 alkyl. Preferably, R₁ is a C1-C4 alkyl, further preferably -CH₂CH₃.

In an optional embodiment of the present application, R₂ is H;

In an optional embodiment of the present application, R₃ is 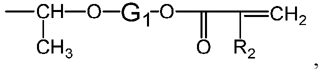 R₂ and G₁ have the same definitions as those in formula (1). In G₁, examples of C1-C12 alkylene include but are not limited to, methylene, ethylene, propylene, butylene, pentylene and 3-methylpentylene. In an optional embodiment of the present application, G₁ is -CH₂CH₂-, -CH₂CH₂CH₂CH₂-, or -CH₂CH₂OCH₂CH₂-.

The present application also provides a benzophenone derivative, wherein the benzophenone derivative has a structure represented by formula (2): 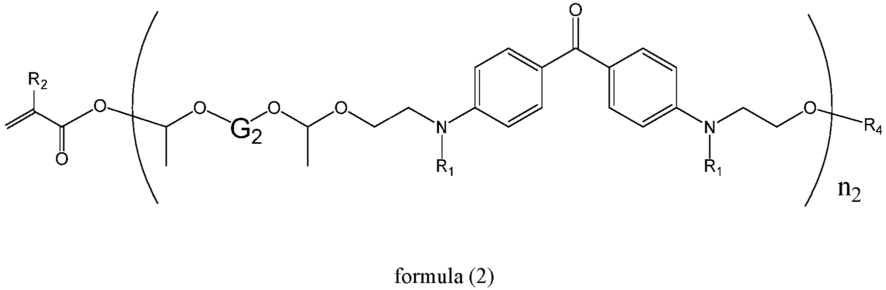 wherein:
n₂ is an integer from 1 to 10, R₁ is an optionally substituted C1-C8 alkyl, R₂ is H or -CH₃,
R₄ is H or 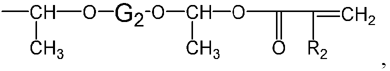
G₂ is an optionally substituted C1-C12 alkylene or 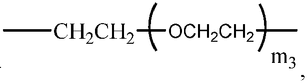 and m₃ is an integer from 1 to 11.

In the present application, n₂ is an integer from 1 to 10, such as 1, 2, 3, 4, 5, 6, 7, 8, 9 or 10. In an optional embodiment of the present application, n₂ is an integer from 1 to 6.

In R₁, examples of C1-C8 alkyls include but are not limited to methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2,2-dimethyl-1-butyl, 3,3-dimethyl-1-butyl, 2-ethyl-1-butyl, n-butyl, isobutyl, sec-butyl, tert-butyl, n-pentyl, isopentyl, neopentyl, etc. In some embodiments, alkyl is C1-C8 alkyl, C1-C7 alkyl, C1-C6 alkyl, C1-C5 alkyl, C1-C4 alkyl, C1-C3 alkyl, C1-C2 alkyl or C1 alkyl. Preferably, R₁ is a C1-C4 alkyl, further preferably -CH₂CH₃.

In an optional embodiment of the present application, R₂ is H;

In an optional embodiment of the present application, R₄ is 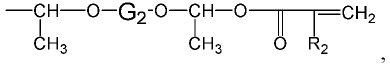 R₂ and G₂ have the same definitions as those in formula (2).

In G₂, examples of C1-C12 alkylene include, but are not limited to, methylene, ethylene, propylene, butylene, pentylene and 3-methylpentylene.

The present application also provides a benzophenone derivative, wherein the benzophenone derivative has a structure represented by formula (3): 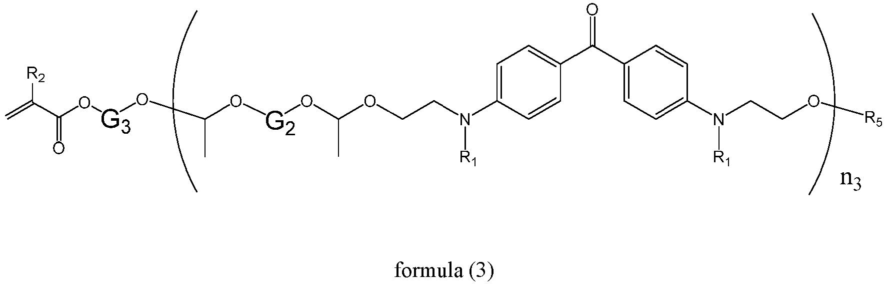 wherein:
n₃ is an integer from 1 to 10, R₁ is an optionally substituted C1-C8 alkyl, R₂ is H or -CH₃, R₅ is H or 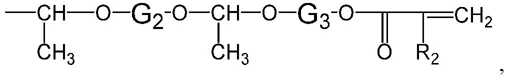
G₂ is an optionally substituted C1-C12 alkylene or 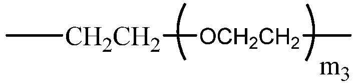 , m₃ is an integer from 1 to 11;
G₃ is an optionally substituted C1-C12 alkylene, 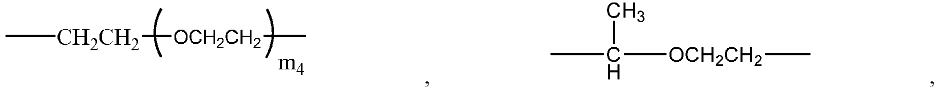 or 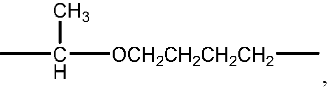 and m₄ is an integer from 1 to 11.

In the present application, n₃ is an integer from 1 to 10, such as 1, 2, 3, 4, 5, 6, 7, 8, 9 or 10. In an optional embodiment of the present application, n₃ is an integer from 1 to 6.

In R₁, examples of C1-C8 alkyls include but are not limited to methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2,2-dimethyl-1-butyl, 3,3-dimethyl-1-butyl, 2-ethyl-1-butyl, n-butyl, isobutyl, sec-butyl, tert-butyl, n-pentyl, isopentyl, neopentyl, etc. In some embodiments, alkyl is C1-C8 alkyl, C1-C7 alkyl, C1-C6 alkyl, C1-C5 alkyl, C1-C4 alkyl, C1-C3 alkyl, C1-C2 alkyl or C1 alkyl. Preferably, R₁ is a C1-C4 alkyl, further preferably -CH₂CH₃.

In an optional embodiment of the present application, R₂ is H;

In an optional embodiment of the present application, R₅ is 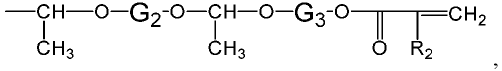 R₂, G₂ and G₃ have the same definitions as those in formula (3). In G₃, examples of C1-C12 alkylene include but are not limited to, methylene, ethylene, propylene, butylene, pentylene and 3-methylpentylene.

In an optional embodiment of the present application, G₂ and G₃ are each independently -CH₂CH₂-, -CH₂CH₂CH₂CH₂-, -CH₂CH₂OCH₂CH₂- or -CH₂CH₂OCH₂CH₂OCH₂CH₂-

The present application also provides a method for preparing the benzophenone derivative having the structure of formula (1) as described above, comprising:
performing a reaction between the compound represented by formula (4) and the compound represented by formula (5) until at least one of the hydroxyl groups at both ends of the compound represented by formula (4) is fully added, and then ending the reaction to obtain a benzophenone derivative with the structure of formula (1); 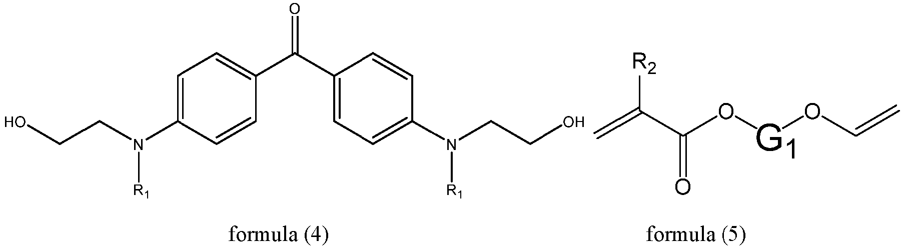
wherein a molar ratio of the compound represented by formula (4) to the compound represented by formula (5) is in a range from 1:1 to 1:2, and R₁, R₂ and G₁ have the same definitions as those in formula (1).

In an optional embodiment of the present application, the reaction temperature is in a range from 40°C to 120°C and the reaction time is in a range from 10h to 100h.

In the above reaction, when at least one of the hydroxyl groups at both ends of the compound represented by formula (4) is fully added, the reaction is completed. The reaction can be carried out under stirring conditions.

In an optional embodiment of the present application, after the reaction is completed, the obtained reaction liquid is washed and the solvent is recovered to obtain a benzophenone derivative with the structure of formula (1).

An exemplary washing method is: first washing with 1-5% dilute weak alkaline aqueous solution, and then washing with water. The weak alkaline aqueous solution can be an aqueous solution of sodium bicarbonate or sodium carbonate or ammonium carbonate.

An exemplary method for recovering the solvent is distillation to recover the solvent.

Optionally, the method for preparing the benzophenone derivative with the above formula (1) structure comprises the following steps:
mixing the compound represented by formula (4), the compound represented by formula (5), a polymerization inhibitor, a catalyst and a solvent to obtain a mixture, heating the mixture under nitrogen or an inert atmosphere for a reaction; lowing the temperature at the end of the reaction, adding an alkaline solution for washing, and then washing with water until neutral; removing the solvent to obtain the benzophenone derivative with the structure of formula (1); 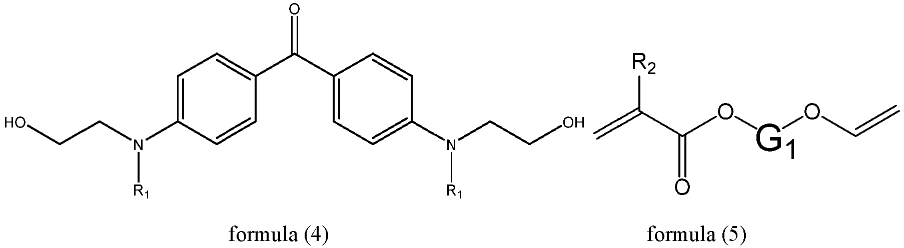
wherein a molar ratio of the compound represented by formula (4) to the compound represented by formula (5) is in a range from 1:1 to 1:2, and R₁, R₂ and G₁ have the same definitions as those in formula (1).

Optionally, the reaction temperature is in a range from 40°C to 120°C and the reaction time is in a range from 10h to 100h. Optionally, it can be detected by HPLC until the compound represented by formula (4) reacts completely and the monoaddition product reacts completely.

Optionally, the polymerization inhibitor is at least one selected from the group consisting of polyphenols, substituted phenol polymerization inhibitors, quinone polymerization inhibitors and diarylamine polymerization inhibitors. Preferably, the polymerization inhibitor is at least one of hydroquinone, p-methoxyphenol, 2,6-di-tert-butylphenol, p-benzoquinone, phenothiazine, or hindered amine oxide, more preferably hydroquinone and/or phenothiazine.

Optionally, the amount of the polymerization inhibitor is 0.001% to 10% of the mass of the compound represented by formula (5), such as 1%, 2%, 3%, 4%, 5%, 6%, 7%, 8 %, 9% or 10%.

Optionally, the catalyst is one or more of organic sulfonic acid, strongly acidic cation exchange resin or organic sulfonates, preferably p-aminobenzene sulfonic acid or poly-4-vinylpyridine p-toluenesulfonic acid salt. The amount of the catalyst is 0.1% to 20% of the mass of the compound represented by formula (4).

Optionally, the solvent is an organic solvent immiscible with water, preferably toluene. The amount of the solvent can be added according to actual needs to ensure that the raw materials are dissolved. Optionally, the amount of the solvent is 0.5-100 times the mass of the compound represented by formula (4).

Optionally, the amount of the alkaline solution added can be 1-100 times the amount of catalyst. The alkaline solution may be a sodium carbonate solution, and the mass concentration of the sodium carbonate solution is 1% to 10%.

Optionally, after the reaction is completed, the temperature is lowered to 20°C to 40°C.

Optionally, the content of effective groups in the benzophenone derivative of formula (1) is 30% to 60%, where the effective group refers to the molecular group

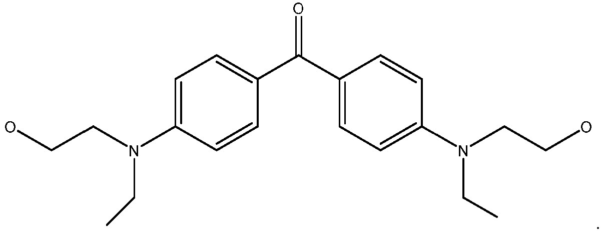

The present application also provides a method for preparing a benzophenone derivative having a structure of formula (2) as described above, comprising:
performing a reaction between the compound represented by formula (6) and the compound represented by formula (7) until at least one of the hydroxyl groups at both ends of the compound represented by formula (6) is fully added, adding the compound represented by formula (8) and continuing to react until the content of the compound represented by equation (8) no longer decreases, and stopping the reaction; 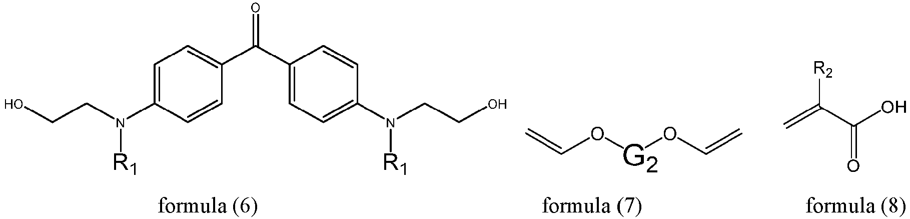
wherein a molar ratio of the compound represented by formula (6) to the compound represented by formula (7) is in a range from 1:1 to 1:2, and a molar ratio of the compound represented by formula (6) to the compound represented by formula (8) is in a range from 1:0.5 to 1:3, and R₁, R₂ and G₂ have the same definitions as those in formula (2).

In an optional embodiment of the present application, the reaction temperature is in a range from 40°C to 120°C and the reaction time is in a range from 10h to 100h.

The reaction can be carried out under stirring conditions.

In an optional embodiment of the present application, after all the double bonds in the vinyl group in the compound represented by formula (8) are added, the reaction is completed, and the resulting reaction liquid is washed and the solvent is recovered to obtain a benzophenone derivatives with the structure of formula (2).

An exemplary washing method is: first washing with a dilute weak alkaline aqueous solution, and then washing with water.

An exemplary method of recovering the solvent is distillation to recover the solvent.

Optionally, the method for preparing the benzophenone derivative with the structure of formula (2) comprises the following steps: mixing the compound represented by formula (6), the compound represented by formula (7), a polymerization inhibitor, a catalyst and a solvent to obtain a mixture, performing a heating reaction under nitrogen or an inert atmosphere; after the reaction is completed, adding the compound represented by formula (8), continuing to carry out the reaction under heat preservation until the content of the compound represented by formula (8) no longer decreasing, stopping the reaction, lowing the temperature, and then adding an alkaline solution for washing, then washing with water until neutral, removing the solvent to obtain the benzophenone derivative with the structure of formula (2); 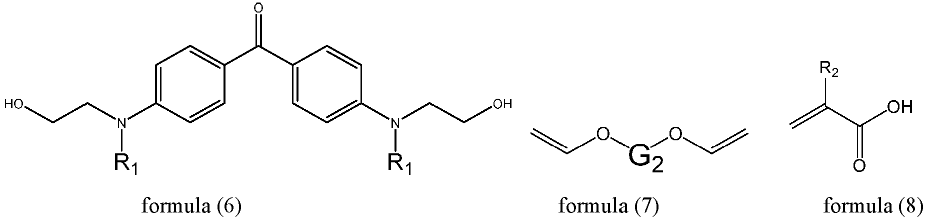 wherein a molar ratio of the compound represented by formula (6) to the compound represented by formula (7) is in a range from 1:1 to 1:2, and a molar ratio of the compound represented by formula (6) to the compound represented by formula (8) is in a range from 1:0.5 to 1:3, and R₁, R₂ and G₂ have the same definitions as those in formula (2).

Optionally, the heating reaction temperature is 40-120°C, and the heating reaction time is 10-100h. Optionally, it can be detected by HPLC until the compound represented by formula (6) reacts completely and the monoaddition product reacts completely.

Optionally, the polymerization inhibitor is at least one selected from the group consisting of polyphenols, substituted phenol polymerization inhibitors, quinone polymerization inhibitors and diarylamine polymerization inhibitors. Preferably, the polymerization inhibitor is at least one of hydroquinone, p-methoxyphenol, 2,6-di-tert-butylphenol, p-benzoquinone, phenothiazine, or hindered amine oxide, more preferably hydroquinone and/or phenothiazine.

Optionally, the amount of polymerization inhibitor is 0.001% to 10% of the mass of the compound represented by formula (7), such as 1%, 2%, 3%, 4%, 5%, 6%, 7%, 8 %, 9% or 10%.

Optionally, the catalyst is one or more of organic sulfonic acid, strongly acidic cation exchange resin or organic sulfonates, preferably p-aminobenzene sulfonic acid or poly-4-vinylpyridine p-toluenesulfonic acid salt. The amount of catalyst is 0.1% to 20% of the mass of the compound represented by formula (6).

Optionally, the solvent is an organic solvent immiscible with water, preferably toluene. The amount of solvent can be added according to actual needs to ensure that the raw materials are dissolved. Optionally, the amount of solvent is 0.5-100 times the mass of the compound shown in formula (6).

Optionally, the amount of alkaline solution added can be 1-100 times the amount of catalyst. The alkaline solution may be a sodium carbonate solution, and the mass concentration of the sodium carbonate solution is 1% to 10%.

Optionally, after the reaction is completed, the temperature is lowered to 20°C to 40°C.

Optionally, the content of effective groups in the benzophenone derivative of formula (2) is 30% to 60%, where the effective group refers to the molecular group

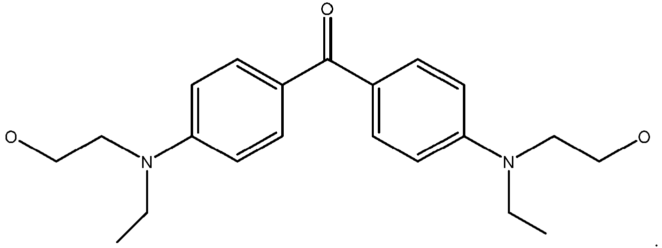

The present application also provides a method for preparing a benzophenone derivative having a structure represented by formula (3) as described above, comprising:
performing a reaction between the compound represented by formula (9) and the compound represented by formula (10) until at least one of the hydroxyl groups at both ends of the compound represented by formula (9) is fully added, then adding the compound represented by formula (11), and continuing to react until all double bonds in the vinyl of the compound represented by formula (11) are added; 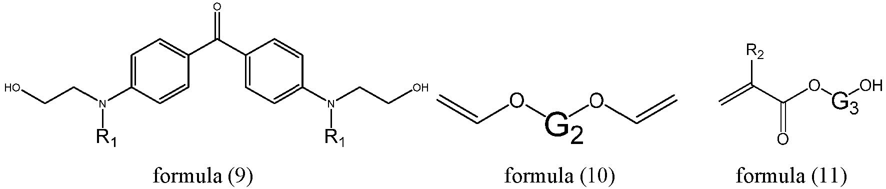
wherein a molar ratio of the compound represented by formula (9) and the compound represented by formula (10) is 1:1 to 1:2, and the molar ratio of the compound represented by formula (9) and the compound represented by formula (11) is 1:1 to 1:3, and R₁, R₂, G₂ and G₃ have the same definitions as those in formula (3).

The reaction can be carried out under stirring conditions.

In an optional embodiment of the present application, the reaction temperature is in a range from 40°C to 120°C and the reaction time is in a range from 10h to 100h.

In an optional embodiment of the present application, after all the double bonds in the vinyl group in the compound represented by formula (11) are added, the reaction is completed, and the resulting reaction liquid is washed and the solvent is recovered to obtain a benzophenone derivatives with the structure of formula (3).

An exemplary washing method is: first washing with a dilute weak alkaline aqueous solution, and then washing with water.

An exemplary method of recovering the solvent is distillation to recover the solvent.

Optionally, the method for preparing the benzophenone derivative with the structure of formula (3) comprises the following steps: mixing the compound represented by formula (9), the compound represented by formula (10), a polymerization inhibitor, a catalyst and a solvent, and performing a heating reaction under nitrogen or an inert atmosphere; after the reaction is completed, adding the compound represented by formula (11),continuing to carry out the reaction under heat preservation until the content of the compound represented by formula (11) no longer decreasing, stopping the reaction, lowing the temperature, adding an alkaline solution for washing, then washing with water until neutral, removing the solvent to obtain the benzophenone derivative with the structure of formula (2); 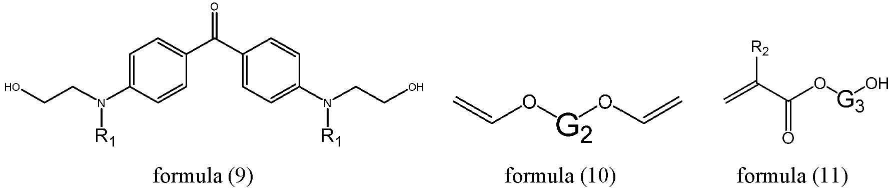 wherein a molar ratio of the compound represented by formula (9) and the compound represented by formula (10) is 1:1 to 1:2, and the molar ratio of the compound represented by formula (9) and the compound represented by formula (11) is 1:1 to 1:3, and R₁, R₂, G₂ and G₃ have the same definitions as those in formula (3).

Optionally, the reaction temperature is in a range from 40°C to 120°C and the reaction time is in a range from 10h to 100h. Optionally, it can be detected by HPLC until the compound represented by formula (9) reacts completely and the monoaddition product reacts completely.

Optionally, the polymerization inhibitor is at least one selected from the group consisting of polyphenols, substituted phenol polymerization inhibitors, quinone polymerization inhibitors and diarylamine polymerization inhibitors. Preferably, the polymerization inhibitor is at least one of hydroquinone, p-methoxyphenol, 2,6-di-tert-butylphenol, p-benzoquinone, phenothiazine, or hindered amine oxide, more preferably hydroquinone and/or phenothiazine. Optionally, the amount of the polymerization inhibitor is 0.001% to 10% of the mass of the compound represented by formula (10), such as 1%, 2%, 3%, 4%, 5%, 6%, 7%, 8 %, 9% or 10%.

Optionally, the catalyst is one or more of organic sulfonic acid, strongly acidic cation exchange resin or organic sulfonates, preferably p-aminobenzene sulfonic acid or poly-4-vinylpyridine p-toluenesulfonic acid salt. The amount of the catalyst is 0.1% to 20% of the mass of the compound represented by formula (9).

Optionally, the solvent is an organic solvent immiscible with water, preferably toluene. The amount of solvent can be added according to actual needs to ensure that the raw materials are dissolved. Optionally, the amount of the solvent is 0.5-100 times the mass of the compound shown in formula (9).

Optionally, the amount of the alkaline solution added can be 1-100 times the amount of catalyst. The alkaline solution may be a sodium carbonate solution, and the mass concentration of the sodium carbonate solution is 1% to 10%. Optionally, after the reaction is completed, the temperature is lowered to 20°C to 40°C.

Optionally, the content of effective groups in the benzophenone derivative of formula (3) is 30% to 60%, where the effective group refers to the molecular group

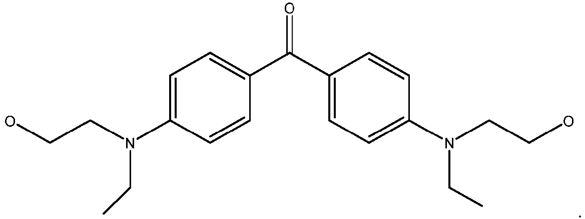

The preparation of the compound represented by formula (1), formula (2) or formula (3) of the present application is preferably carried out in the presence of a polymerization inhibitor, a catalyst and a solvent.

Optionally, the polymerization inhibitor is at least one selected from the group consisting of polyphenols, substituted phenol polymerization inhibitors, quinone polymerization inhibitors and diarylamine polymerization inhibitors. Preferably, the polymerization inhibitor is at least one of hydroquinone, p-methoxyphenol, 2,6-di-tert-butylphenol, p-benzoquinone, phenothiazine, or hindered amine oxide, more preferably hydroquinone and/or phenothiazine. Optionally, the amount of the polymerization inhibitor is 0.001% to 10% of the mass of the compound represented by formula (5), formula (8) or formula (11), such as 1%, 2%, 3%, 4%, 5%, 6%, 7%, 8 %, 9% or 10%.

Optionally, the catalyst is one or more of organic sulfonic acid, strongly acidic cation exchange resin or organic sulfonates, preferably p-aminobenzene sulfonic acid or poly-4-vinylpyridine p-toluenesulfonic acid salt. The amount of the catalyst is 0.1% to 20% of the mass of the compound represented by formula (4), formula (6) or formula (9).

Optionally, the solvent is an organic solvent immiscible with water, preferably toluene.

According to another aspect of the present application, a photoinitiator composition is provided, wherein the photoinitiator composition comprises a photoinitiator for free radical polymerization and the benzophenone derivative as described above.

According to another aspect of the present application, a photocurable composition is provided, wherein the photocurable composition comprises a photoinitiator component and a radically polymerizable ethylenically unsaturated compound, wherein the photoinitiator component comprises the photoinitiator composition as described above.

In an optional embodiment of the present application, the photocurable composition comprises: (a) the benzophenone derivative as described above; (b) a photoinitiator for free radical polymerization; and (c) a free-radically polymerizable ethylenically unsaturated compound.

The photocurable composition including the aforementioned photoinitiator composition has low mobility.

In an optional embodiment of the present application, the component (a) is added in an amount ranging from from 0.1% to 20% of the total weight of the photocurable composition, such as 1%, 5%, 10%, 15% or 20%.

In an optional embodiment of the present application, the component (b) is a commonly used compound in the industry that is commercially or laboratory-available, and is selected from the group consisting of benzophenones (except benzophenones and derivatives other than those in the present application), thioxanthone compounds, α-hydroxyketone compounds, α-aminoketones compounds, acyl phosphine oxide compounds, oxime lipid compounds, and any combination thereof; preferably, at least one of benzophenone, 2-isopropylthiaxanthone, 2-dimethylamino-2-(4-methyl benzyl)-1-(4-morpholinylphenyl)-1-butanone, 2,4,6-trimethylbenzoyl-diphenylphosphine oxide, photoinitiator Omnipol TX, photoinitiator Omnipol 910 and photoinitiator Omnipol TP. Optionally, the photoinitiator Omnipol TX, photoinitiator Omnipol 910 or photoinitiator Omnipol TP is selected from the macromolecular photoinitiator series products Omnipol TX, Omnipol 910 or Omnipol TP of IGM Resin Company.

In an optional embodiment of the present application, the component (b) is added in an amount ranging from 0.1% to 10% of the total weight of the photocurable composition, such as 0.1%, 2%, 4%, 6%, 8% or 10%.

Ethylenically unsaturated compounds represent ethylenically unsaturated monomers, oligomers, prepolymers and mixtures thereof, which are capable of free radical polymerization.

In an optional embodiment of the present application, the component (c) is at least one selected from the group consisting of epoxy acrylate resin, polyurethane acrylate resin, polyester acrylate resin, polyether acrylate resin, and acrylate resin, polyacrylate, epoxy methacrylate resin, polyurethane methacrylate resin, polyester methacrylate resin, polyether methacrylate resin, acrylated polymethacrylate, allyl ether compound, acrylate monomer and methacrylate monomer. The acrylate monomer or methacrylate monomer is independently monofunctional, difunctional or polyfunctional. These free-radically polymerizable ethylenically unsaturated compounds are readily available commercially or in the laboratory to those skilled in the art.

The photocurable composition may also contain other additives to meet performance requirements, such as pigments, fillers, leveling aids, polymerization inhibitors, solvents, etc.

According to another aspect of the present application, there is provided a use of the photocurable composition as described above in food packaging printing, pharmaceutical packaging printing, furniture coating, book printing and advertising printing.

According to another aspect of the present application, a photocurable product is provided, which is formed by photocuring a photocurable composition, wherein the photocurable composition is the photocurable composition as described above, preferably, the photocurable product is selected from any one of coatings, adhesives, and printing inks.

According to another aspect of the present application, a method for curing a photocurable composition is provided, comprising coating the photocurable composition as described above on a substrate; and curing the photocurable composition by using a light source with an emission band in the UV-visible light region.

The substrate is selected from the group consisting of wood, paper, plastic, coating and metal. The coating method is selected from the group consisting of offset printing, gravure printing, flexographic printing, inkjet printing and 3D printing.

Optionally, after coating on the substrate, the photocurable composition is cured by irradiating with UV-visible light having a wavelength ranging from 200 nm to 425 nm, preferably the photocurable composition is cured by irradiating with UV-visible light having a wavelength ranging from 365 nm to 405 nm.

Beneficial effects:
Compared with EMK, the bis(dialkylamino) benzophenone compound with an acrylate group side chain provided by the present application has a remarkably increased molecular weight and contains a polymerizable double bond, showing remarkably lower mobility.

The benzophenone derivative can be used as an important co-initiator in UV light-curing formulas to initiate photopolymerization of unsaturated carbon-carbon double bond compounds together with other photoinitiators. The compound has very low mobility due to its large molecular weight and is suitable to replace N,N,N,N-tetraethyl-4,4'-diaminobenzophenone in the fields of food packaging, printing formulations, and the like.

### DETAILED DESCRIPTION

Experimental raw materials and materials:

| Reagents Name | Reagent specifications and content | Reagent source |
|---|---|---|
| HEMK* | >98% | Self made, reference: CN107434773A |
| Butyl glycidyl ether | >98% | Shanghai Aladdin Bio-Chem Technology Co., LTD |
| Ethyl glycidyl ether | >98% | Shanghai Aladdin Bio-Chem Technology Co., LTD |
| Ethylene oxide | >98% | Shanghai Aladdin Bio-Chem Technology Co., LTD |
| Propylene oxide | >98% | Shanghai Aladdin Bio-Chem Technology Co., LTD |
| Potassium hydroxide | AR | Shanghai Aladdin Bio-Chem Technology Co., LTD |
| Sodium hydroxide | AR | Shanghai Aladdin Bio-Chem Technology Co., LTD |
| Anhydrous sodium carbonate | AR | Shanghai Aladdin Bio-Chem Technology Co., LTD |
| Triethylamine | AR | Shanghai Aladdin Bio-Chem Technology Co., LTD |
| Acetic Anhydride | >98% | Shanghai Aladdin Bio-Chem Technology Co., LTD |
| Acryloyl chloride | >98% | Shanghai Aladdin Bio-Chem Technology Co., LTD |
| Butyl isocyanate | >98% | Shanghai Aladdin Bio-Chem Technology Co., LTD |
| 2-acryloyloxyethyl isocyanate | >98% | Shanghai CAB New Materials Technology Co., Ltd |

| | | |
|---|---|---|
| *The structure of HEMK is 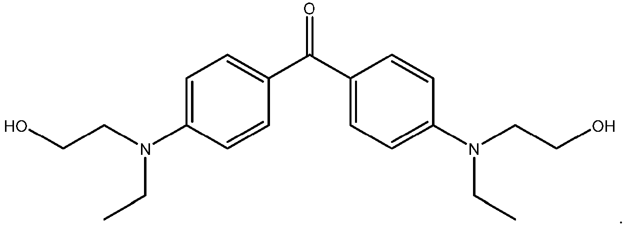 Omnipol TX is a photoinitiator of polybutylene glycol 250-bis(2-carboxymethoxythioxanthone) ester, a product of IGM RESINS; Omnirad EMK is 4,4'-bis(diethylamino)benzophenone, a product of IGM RESINS; Photomer 4072 is trimethylolpropane propoxy (3) triacrylate, a product of IGM RESINS; Photomer 3316 is a low viscosity modified epoxy acrylate, a product of IGM RESINS. | | |

### Example 1

To a 100mL three-necked flask with mechanical stirrer, 1.78g (5mmol) of HEMK, 1.86g (10mmol) of 2-vinyloxyethoxyethyl acrylate (VEEA), 0.022g (0.2mmol) of hydroquinone, 0.086g (0.5mmol) of p-aminobenzenesulfonic acid and 12.0g of toluene were added in sequence. After sufficient nitrogen replacement under room temperature with stirring, the mixture was heated and stirred for reaction under the nitrogen balloon sealing. The reaction temperature was 55°C and the reaction time was 48 hours. A sample was taken for HPLC detection and the reaction was stopped until HEMK was complete reacted and the reaction of monoaddition product was completed. The temperature was lowered to 30°C, 3.0g sodium carbonate aqueous solution with a mass concentration of 5% (1.4mmol sodium carbonate) was added, and then washed until neutral. The solvent was distilled off under reduced pressure to obtain 3.44g of the product of formula IX, with an effective group content of 55%. The effective group refers to the remaining residue in the HEMK structure after removing the hydrogen on the two hydroxyl groups.

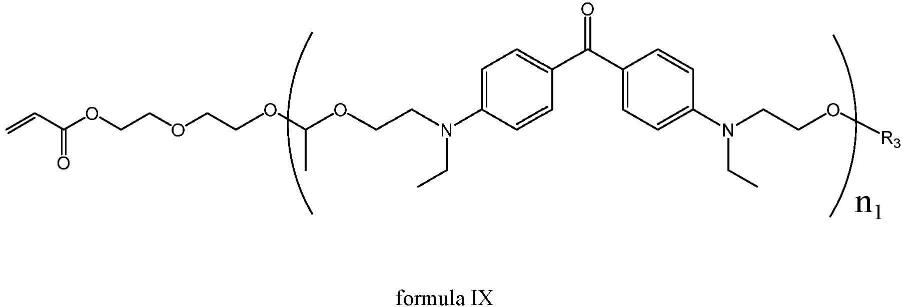

**Table 1 Analysis results of the product of Example 1 by liquid chromatography-mass spectrometry**

| Number | Retention time (min) | Content (%) | Molecular mass | Value of n₁ in formula IX | R₃ in formula IX |
|---|---|---|---|---|---|
| 1 | 3.16 | 1.87 | 542.67 | 1 | H |
| 2 | 5.87 | 40.36 | 728.88 | 1 | 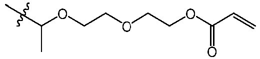 |
| 3 | 7.07 | 3.34 | 925.18 | 2 | H |
| 4 | 10.19 | 24.27 | 1111.38 | 2 | 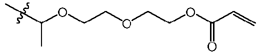 |
| 5 | 10.94 | 2.05 | 1307.68 | 3 | H |
| 6 | 13.08 | 15.08 | 1493.89 | 3 | 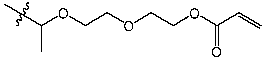 |
| 7 | 13.56 | 1.20 | 1690.19 | 4 | H |
| 8 | 14.99 | 6.25 | 1876.39 | 4 | 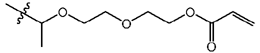 |
| 9 | 15.49 | 0.37 | 2072.7 | 5 | H |
| 10 | 16.94 | 3.26 | 2258.89 | 5 | 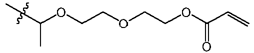 |
| 11 | 19.52 | 1.31 | 2455.21 | 6 | H |
| 12 | 23.14 | 0.54 | 2641.39 | 6 | 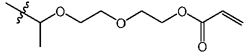 |

### Example 2

To a 100mL three-necked flask with mechanical stirrer, 1.78g (5mmol) of HEMK, 1.86g (10mmol) of 2-vinyloxyethoxyethyl acrylate (VEEA), 0.022g (0.2mmol) of hydroquinone, 0.086g (0.11mmol) of poly(4-vinylpyridine-p-toluenesulfonate) and 12.0g of toluene were added in sequence. After sufficient nitrogen replacement under room temperature with stirring, the mixture was heated and stirred for reaction under the nitrogen balloon sealing. The reaction temperature was 55°C and the reaction time was 48 hours. A sample was taken for HPLC detection and the reaction was stopped until HEMK was complete reacted and the reaction of monoaddition product was completed. The temperature was lowered to 30°C, 3.0g sodium carbonate aqueous solution with a mass concentration of 5% (1.4mmol sodium carbonate) was added, and then washed until neutral. The solvent was distilled off under reduced pressure to obtain 3.40g of the product of formula IX.

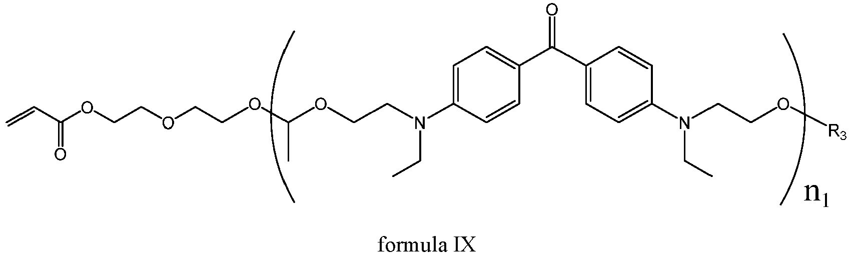

**Table 2 Analysis results of the product of Example 2 by liquid chromatography-mass spectrometry**

| Number | Retention time (min) | Content (%) | Molecular mass | Value of n₁ in formula IX | R₃ in formula IX |
|---|---|---|---|---|---|
| 1 | 3.16 | 7.29 | 542.67 | 1 | H |
| 2 | 5.87 | 28.74 | 728.88 | 1 | 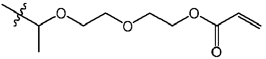 |
| 3 | 7.07 | 5.32 | 925.18 | 2 | H |
| 4 | 10.19 | 24.57 | 1111.38 | 2 | 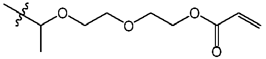 |
| 5 | 10.94 | 2.85 | 1307.68 | 3 | H |
| 6 | 13.08 | 15.92 | 1493.89 | 3 | 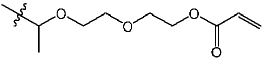 |
| 7 | 13.56 | 1.38 | 1690.19 | 4 | H |
| 8 | 14.99 | 7.75 | 1876.39 | 4 | 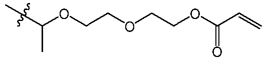 |
| 9 | 15.49 | 0.42 | 2072.7 | 5 | H |
| 10 | 16.94 | 3.87 | 2258.89 | 5 | 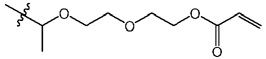 |
| 11 | 19.52 | 1.00 | 2455.21 | 6 | H |
| 12 | 23.14 | 0.88 | 2641.39 | 6 | 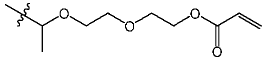 |

### Example 3

To a 100mL three-necked flask with mechanical stirrer, 1.78g (5mmol) of HEMK, 2.00g (10mmol) of 2-vinyloxyethoxyethyl methacrylate (VEEM), 0.022g (0.2mmol) of hydroquinone, 0.086g (0.5mmol) of p-aminobenzenesulfonic acid and 12.0g of toluene were added in sequence. After sufficient nitrogen replacement under room temperature with stirring, the mixture was heated and stirred for reaction under the nitrogen balloon sealing. The reaction temperature was 55°C and the reaction time was 48 hours. A sample was taken for HPLC detection and the reaction was stopped until HEMK was complete reacted and the reaction of monoaddition product was completed. The temperature was lowered to 30°C, 3.0g sodium carbonate aqueous solution with a mass concentration of 5% (1.4mmol sodium carbonate) was added, and then washed until neutral. The solvent was distilled off under reduced pressure to obtain 3.51g of the product of formula X.

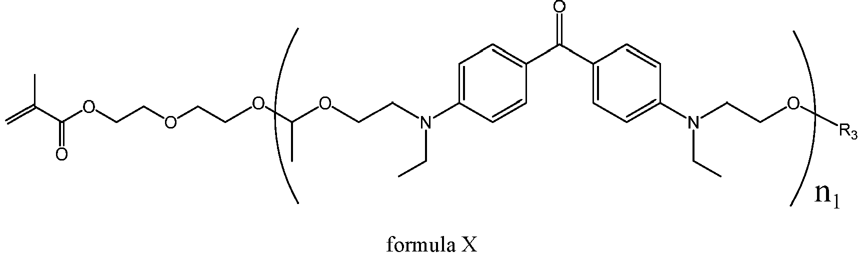

**Table 3 Analysis results of the product of Example 3 by liquid chromatography-mass spectrometry**

| Number | Retention time (min) | Content (%) | Molecular mass | Value of n₁ in formula X | R₃ in formula X |
|---|---|---|---|---|---|
| 1 | 3.12 | 2.58 | 556.69 | 1 | H |
| 2 | 5.80 | 38.69 | 756.92 | 1 | 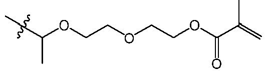 |
| 3 | 6.92 | 3.6 | 939.20 | 2 | H |
| 4 | 10.03 | 25.21 | 1139.42 | 2 | 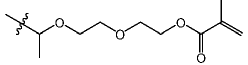 |
| 5 | 10.88 | 2.39 | 1321.70 | 3 | H |
| 6 | 12.99 | 16.23 | 1521.93 | 3 | 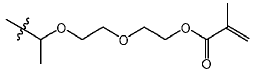 |
| 7 | 13.32 | 1.27 | 1704.21 | 4 | H |
| 8 | 14.58 | 5.34 | 1904.43 | 4 | 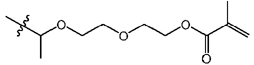 |
| 9 | 15.31 | 0.38 | 2086.72 | 5 | H |
| 10 | 16.72 | 2.85 | 2286.93 | 5 | 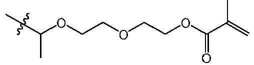 |
| 11 | 18.98 | 1.03 | 2469.23 | 6 | H |
| 12 | 22.34 | 0.40 | 2669.43 | 6 | 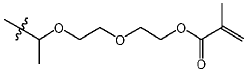 |

### Example 4

To a 100mL three-necked flask with mechanical stirrer, 1.78g (5mmol) of HEMK, 1.42g (10mmol) of 2-vinyloxyethyl acrylate (VEA), 0.022g (0.2mmol) of hydroquinone, 0.086g (0.5mmol) of p-aminobenzenesulfonic acid and 12.0g of toluene were added in sequence. After sufficient nitrogen replacement under room temperature with stirring, the mixture was heated and stirred for reaction under the nitrogen balloon sealing. The reaction temperature was 55°C and the reaction time was 48 hours. A sample was taken for HPLC detection and the reaction was stopped until HEMK was complete reacted and the reaction of monoaddition product was completed. The temperature was lowered to 30°C, 3.0g sodium carbonate aqueous solution with a mass concentration of 5% (1.4mmol sodium carbonate) was added, and then washed until neutral. The solvent was distilled off under reduced pressure to obtain 2.91g of the product of formula XI, with an effective group content of 55%.

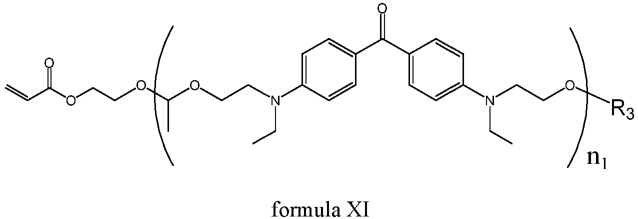

**Table 4 Analysis results of the product of Example 4 by liquid chromatography-mass spectrometry**

| Number | Retention time (min) | Content (%) | Molecular mass | Value of n₁ in formula XI | R₃ in formulaXI |
|---|---|---|---|---|---|
| 1 | 3.02 | 1.0 6 | 498.62 | 1 | H |
| 2 | 5.44 | 40. 58 | 640.77 | 1 | 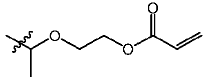 |
| 3 | 6.85 | 1.5 8 | 881.12 | 2 | H |
| 4 | 9.76 | 25. 84 | 1023.27 | 2 | 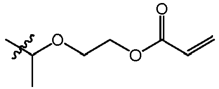 |
| 5 | 10.14 | 1.3 6 | 1264.62 | 3 | H |
| 6 | 12.56 | 15. 21 | 1406.77 | 3 | 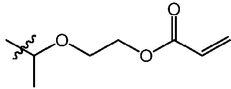 |
| 7 | 13.01 | 0.9 8 | 1647.12 | 4 | H |
| 8 | 14.25 | 7.6 5 | 1789.27 | 4 | 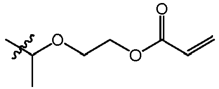 |
| 9 | 14.93 | 0.3 8 | 2029.72 | 5 | H |
| 10 | 15.19 | 2.8 5 | 2171.77 | 5 | 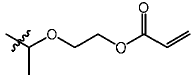 |
| 11 | 17.32 | 1.6 7 | 2412.22 | 6 | H |
| 12 | 20.87 | 0.8 2 | 2554.27 | 6 | 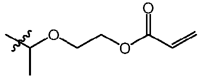 |

### Example 5

To a 100mL three-necked flask with mechanical stirrer, 1.78g (5mmol) of HEMK, 1.42g (10mmol) of 2-vinyloxyethyl acrylate (VEA), 0.022g (0.2mmol) of hydroquinone, 0.086g (0.5mmol) of p-toluenesulfonic acid and 12.0g of dichloroethane were added in sequence. After sufficient nitrogen replacement under room temperature with stirring, the mixture was heated and stirred for reaction under the nitrogen balloon sealing. The reaction temperature was 55°C and the reaction time was 48 hours. A sample was taken for HPLC detection and the reaction was stopped until HEMK was complete reacted and the reaction of monoaddition product was completed. The temperature was lowered to 30°C, 3.0g sodium carbonate aqueous solution with a mass concentration of 5% (1.4mmol sodium carbonate) was added, and then washed until neutral. The solvent was distilled off under reduced pressure to obtain 2.88g of the product of formula XI.

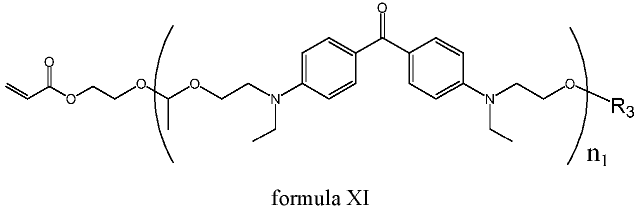

**Table 5 Analysis results of the product of Example 5 by liquid chromatography-mass spectrometry**

| Number | Retention time (min) | Content (%) | Molecular mass | Value of n₁ in formula XI | R₃ in formula XI |
|---|---|---|---|---|---|
| 1 | 3.02 | 0.98 | 498.62 | 1 | H |
| 2 | 5.44 | 36.8 6 | 640.77 | 1 | 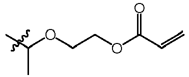 |
| 3 | 6.85 | 2.41 | 881.12 | 2 | H |
| 4 | 9.76 | 24.8 0 | 1023.27 | 2 | 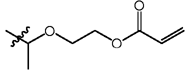 |
| 5 | 10.14 | 1.60 | 1264.62 | 3 | H |
| 6 | 12.56 | 12.8 7 | 1406.77 | 3 | 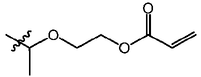 |
| 7 | 13.01 | 1.28 | 1647.12 | 4 | H |
| 8 | 14.25 | 11.5 9 | 1789.27 | 4 | 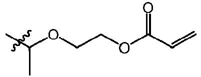 |
| 9 | 14.93 | 0.88 | 2029.72 | 5 | H |
| 10 | 15.19 | 3.81 | 2171.77 | 5 | 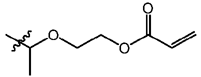 |
| 11 | 17.32 | 1.59 | 2412.22 | 6 | H |
| 12 | 20.87 | 1.32 | 2554.27 | 6 | 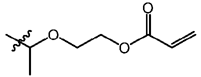 |

### Example 6

To a 100mL three-necked flask with mechanical stirrer, 1.78g (5mmol) of HEMK, 1.56g (12mmol) of 2-vinyloxyethyl methacrylate (VEM), 0.022g (0.2mmol) of hydroquinone, 0.086g (0.5mmol) of p-aminobenzenesulfonic acid and 12.0g of toluene were added in sequence. After sufficient nitrogen replacement under room temperature with stirring, the mixture was heated and stirred for reaction under the nitrogen balloon sealing. The reaction temperature was 55°C and the reaction time was 48 hours. A sample was taken for HPLC detection and the reaction was stopped until HEMK was complete reacted and the reaction of monoaddition product was completed. The temperature was lowered to 30°C, 3.0g sodium carbonate aqueous solution with a mass concentration of 5% (1.4mmol sodium carbonate) was added, and then washed until neutral. The solvent was distilled off under reduced pressure to obtain 3.07g of the product of formula XII.

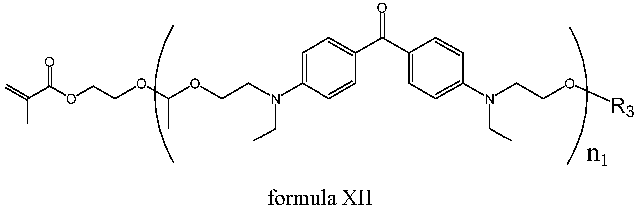

**Table 6 Analysis results of the product of Example 6 by liquid chromatography-mass spectrometry**

| Number | Retention time (min) | Content (%) | Molecul ar mass | Value of n₁ in formula XII | R₃ in formula XII |
|---|---|---|---|---|---|
| 1 | 3.15 | 1.38 | 512.64 | 1 | H |
| 2 | 5.94 | 39.89 | 654.79 | 1 | 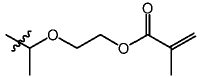 |
| 3 | 7.26 | 1.20 | 895.14 | 2 | H |
| 4 | 10.55 | 26.66 | 1037.29 | 2 | 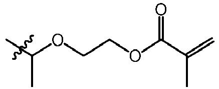 |
| 5 | 10.94 | 1.28 | 1278.64 | 3 | H |
| 6 | 13.20 | 16.38 | 1420.79 | 3 | 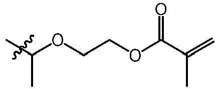 |
| 7 | 14.32 | 0.70 | 1661.14 | 4 | H |
| 8 | 15.00 | 7.49 | 1803.29 | 4 | 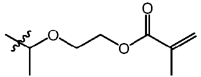 |
| 9 | 15.93 | 0.78 | 2043.64 | 5 | H |
| 10 | 17.49 | 2.42 | 2185.79 | 5 | 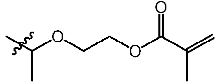 |
| 11 | 19.32 | 1.32 | 2426.14 | 6 | H |
| 12 | 21.87 | 0.49 | 2568.29 | 6 | 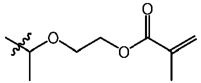 |

### Example 7

To a 100mL three-necked flask with mechanical stirrer, 1.78g (5mmol) of HEMK, 1.58g (10mmol) of diethylene glycol divinyl ether, 0.022g (0.2mmol) of hydroquinone, 0.086g (0.5mmol) of p-aminobenzenesulfonic acid and 12.0g of toluene were added in sequence. After sufficient nitrogen replacement under room temperature with stirring, the mixture was heated and stirred for reaction under the nitrogen balloon sealing. The reaction temperature was 55°C and the reaction time was 48 hours. A sample was taken for HPLC detection and the reaction was stopped until HEMK was complete reacted and the reaction of monoaddition product was completed. Then 0.86g (12mmol) of acrylic acid was added, and continue to stir for reaction under heat preservation. A sample was taken for testing every 4 hours of reaction time. The reaction was stopped until the acrylic acid content no longer decreases. The temperature was lowered to 30°C, 6.4g sodium carbonate aqueous solution with a mass concentration of 5% (3.0mmol sodium carbonate) was added, and then washed until neutral. The solvent was distilled off under reduced pressure to obtain 3.91g of the product of formula XIII, with an effective group content of 40%.

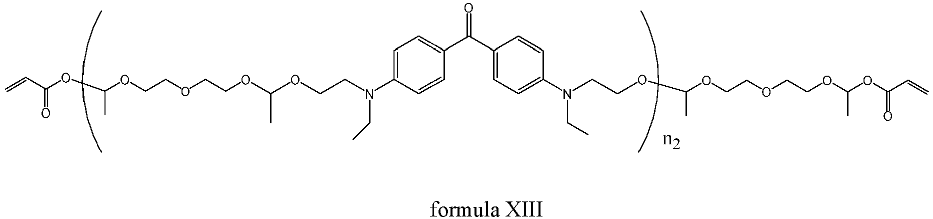

**Table 7 Analysis results of the product of Example 7 by liquid chromatography-mass spectrometry**

| Number | Retention time (min) | Content (%) | Molecular mass | Value of n₂ in formula XIII |
|---|---|---|---|---|
| 1 | 7.20 | 19.44 | 816.99 | 1 |
| 2 | 11.17 | 20.86 | 1331.66 | 2 |
| 3 | 13.92 | 16.51 | 1846.24 | 3 |
| 4 | 15.67 | 12.89 | 2360.91 | 4 |
| 5 | 17.88 | 9.03 | 2875.58 | 5 |
| 6 | 19.76 | 8.24 | 3390.25 | 6 |
| 7 | 22.80 | 5.33 | 3904.92 | 7 |
| 8 | 25.93 | 3.66 | 4419.59 | 8 |
| 9 | 27.62 | 2.58 | 4934.26 | 9 |
| 10 | 30.77 | 1.55 | 5448.93 | 10 |

### Example 8

To a 100mL three-necked flask with mechanical stirrer, 1.78g (5mmol) of HEMK, 0.99g (6.25mmol) of diethylene glycol divinyl ether, 0.022g (0.2mmol) of hydroquinone, 0.086g (0.5mmol) of p-aminobenzenesulfonic acid and 12.0g of toluene were added in sequence. After sufficient nitrogen replacement under room temperature with stirring, the mixture was heated and stirred for reaction under the nitrogen balloon sealing. The reaction temperature was 55°C and the reaction time was 72 hours. A sample was taken for HPLC detection and the reaction was stopped until HEMK was complete reacted and the reaction of monoaddition product was completed. Then 0.22g (3mmol) of acrylic acid was added, and continued to stir for reaction under heat preservation. A sample was taken for testing every 4 hours of reaction time. The reaction was stopped until the acrylic acid content no longer decreases. The temperature was lowered to 30°C, 6.4g sodium carbonate aqueous solution with a mass concentration of 5% (3.0mmol sodium carbonate) was added, and then washed until neutral. The solvent was distilled off under reduced pressure to obtain 2.58g of the product of formula XIII.

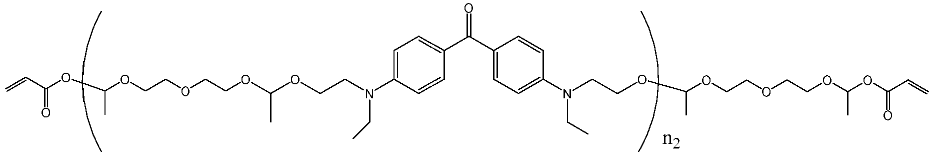

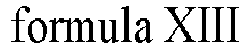

**Table 8 Analysis results of the product of Example 8 by liquid chromatography-mass spectrometry**

| Number | Retention time (min) | Content (%) | Molecular mass | Value of n₂ in formula XIII |
|---|---|---|---|---|
| 1 | 7.20 | 17.15 | 816.99 | 1 |
| 2 | 11.17 | 8.06 | 1331.66 | 2 |
| 3 | 13.92 | 14.51 | 1846.24 | 3 |
| 4 | 15.67 | 14.40 | 2360.91 | 4 |
| 5 | 17.88 | 16.04 | 2875.58 | 5 |
| 6 | 19.76 | 13.04 | 3390.25 | 6 |
| 7 | 22.80 | 8.82 | 3904.92 | 7 |
| 8 | 25.93 | 5.30 | 4419.59 | 8 |
| 9 | 27.62 | 2.05 | 4934.26 | 9 |
| 10 | 30.77 | 0.62 | 5448.93 | 10 |

### Example 9

To a 100mL three-necked flask with mechanical stirrer, 1.78g (5mmol) of HEMK, 1.58g (10mmol) of diethylene glycol divinyl ether, 0.022g (0.2mmol) of hydroquinone, 0.086g (0.5mmol) of p-aminobenzenesulfonic acid and 12.0g of toluene were added in sequence. After sufficient nitrogen replacement under room temperature with stirring, the mixture was heated and stirred for reaction under the nitrogen balloon sealing. The reaction temperature was 55°C and the reaction time was 48 hours. A sample was taken for HPLC detection and the reaction was stopped until HEMK was complete reacted and the reaction of monoaddition product was completed. Then 1.39g (12mmol) of hydroxyethyl acrylate was added, and continued to stir for reaction under heat preservation. A sample was taken for testing every 4 hours of reaction time. The reaction was stopped until the hydroxyethyl acrylate content no longer decreases. The temperature was lowered to 30°C, 6.4g sodium carbonate aqueous solution with a mass concentration of 5% (3.0mmol sodium carbonate) was added, and then washed until neutral. The solvent was distilled off under reduced pressure to obtain 4.15g of the product of formula XIV, with an effective group content of 40%.

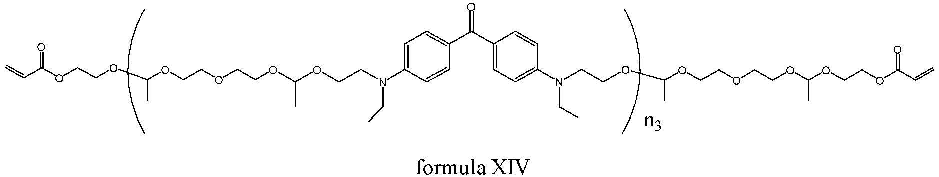

**Table 9 Analysis results of the product of Example 9 by liquid chromatography-mass spectrometry**

| Number | Retention time (min) | Content (%) | Molecular mass | Value of n₃ in formula XIV |
|---|---|---|---|---|
| 1 | 7.28 | 20.54 | 905.09 | 1 |
| 2 | 11.38 | 21.85 | 1419.76 | 2 |
| 3 | 14.00 | 17.06 | 1934.43 | 3 |
| 4 | 15.96 | 12.44 | 2449.10 | 4 |
| 5 | 18.04 | 9.06 | 2963.77 | 5 |
| 6 | 20.39 | 7.92 | 3478.44 | 6 |
| 7 | 23.09 | 4.83 | 3993.11 | 7 |
| 8 | 26.60 | 3.55 | 4507.78 | 8 |
| 9 | 28.51 | 2.18 | 5022.45 | 9 |
| 10 | 31.63 | 1.55 | 5537.12 | 10 |

### Example 10

To a 100mL three-necked flask with mechanical stirrer, 1.78g (5mmol) of HEMK, 0.99g (6.25mmol) of diethylene glycol divinyl ether, 0.022g (0.2mmol) of hydroquinone, 0.086g (0.5mmol) of p-aminobenzenesulfonic acid and 12.0g of toluene were added in sequence. After sufficient nitrogen replacement under room temperature with stirring, the mixture was heated and stirred for reaction under the nitrogen balloon sealing. The reaction temperature was 55°C and the reaction time was 72 hours. A sample was taken for HPLC detection and the reaction was stopped until HEMK was complete reacted and the reaction of monoaddition product was completed. Then 0.35g (3mmol) of hydroxyethyl acrylate was added, and continued to stir for reaction under heat preservation. A sample was taken for testing every 4 hours of reaction time. The reaction was stopped until the hydroxyethyl acrylate content no longer decreases. The temperature was lowered to 30°C, 6.4g sodium carbonate aqueous solution with a mass concentration of 5% (3.0mmol sodium carbonate) was added, and then washed until neutral. The solvent was distilled off under reduced pressure to obtain 2.74g of the product of formula XIV.

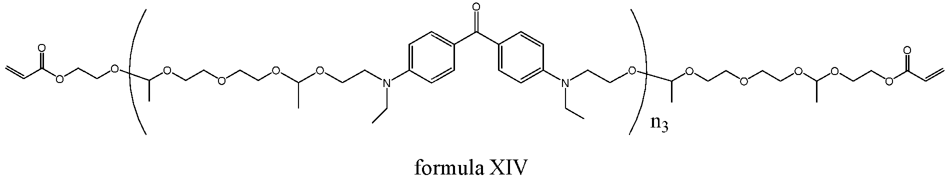

**Table 10 Analysis results of the product of Example 10 by liquid chromatography-mass spectrometry**

| Number | Retention time (min) | Content (%) | Molecular mass | Value of n₃ in formula XIV |
|---|---|---|---|---|
| 1 | 7.28 | 17.15 | 905.09 | 1 |
| 2 | 11.38 | 8.06 | 1419.76 | 2 |
| 3 | 14.00 | 14.51 | 1934.43 | 3 |
| 4 | 15.96 | 14.43 | 2449.10 | 4 |
| 5 | 18.04 | 16.24 | 2963.77 | 5 |
| 6 | 20.39 | 11.79 | 3478.44 | 6 |
| 7 | 23.09 | 7.76 | 3993.11 | 7 |
| 8 | 26.60 | 5.34 | 4507.78 | 8 |
| 9 | 28.51 | 3.19 | 5022.45 | 9 |
| 10 | 31.63 | 1.51 | 5537.12 | 10 |

### Example 11

To a 100mL three-necked flask with mechanical stirrer, 1.78g (5mmol) of HEMK, 2.02g (10mmol) of triethylene glycol divinyl ether, 0.022g (0.2mmol) of hydroquinone, 0.086g (0.5mmol) of p-aminobenzenesulfonic acid and 12.0g of toluene were added in sequence. After sufficient nitrogen replacement under room temperature with stirring, the mixture was heated and stirred for reaction under the nitrogen balloon sealing. The reaction temperature was 55°C and the reaction time was 48 hours. A sample was taken for HPLC detection and the reaction was stopped until HEMK was complete reacted and the reaction of monoaddition product was completed. Then 1.39g (12mmol) of hydroxyethyl acrylate was added, and continued to stir for reaction under heat preservation. A sample was taken for testing every 4 hours of reaction time. The reaction was stopped until the hydroxyethyl acrylate content no longer decreases. The temperature was lowered to 30°C, 6.4g sodium carbonate aqueous solution with a mass concentration of 5% (3.0mmol sodium carbonate) was added, and then washed until neutral. The solvent was distilled off under reduced pressure to obtain 4.66g of the product of formula XV, with an effective group content of 40%.

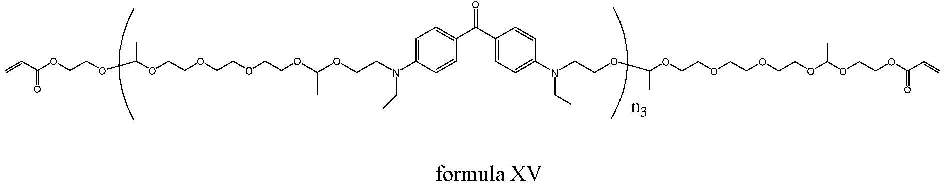

**Table 11 Analysis results of the product of Example 11 by liquid chromatography-mass spectrometry**

| Number | Retention time (min) | Content (%) | Molecular mass | Value of n₃ in formula XV |
|---|---|---|---|---|
| 1 | 7.45 | 21.42 | 993.20 | 1 |
| 2 | 11.83 | 22.03 | 1551.91 | 2 |
| 3 | 15.52 | 17.45 | 2110.63 | 3 |
| 4 | 1677 | 12.33 | 2669.35 | 4 |
| 5 | 18.91 | 8.60 | 3228.07 | 5 |
| 6 | 21.16 | 7.61 | 3786.79 | 6 |
| 7 | 24.42 | 4.38 | 4345.51 | 7 |
| 8 | 27.88 | 3.11 | 4904.23 | 8 |
| 9 | 29.33 | 2.08 | 5462.95 | 9 |
| 10 | 33.55 | 0.98 | 6021.67 | 10 |

### Example 12

To a 100mL three-necked flask with mechanical stirrer, 1.78g (5mmol) of HEMK, 1.26g (6.25mmol) of triethylene glycol divinyl ether, 0.022g (0.2mmol) of hydroquinone, 0.086g (0.5mmol) of p-aminobenzenesulfonic acid and 12.0g of toluene were added in sequence. After sufficient nitrogen replacement under room temperature with stirring, the mixture was heated and stirred for reaction under the nitrogen balloon sealing. The reaction temperature was 55°C and the reaction time was 96 hours. A sample was taken for HPLC detection and the reaction was stopped until HEMK was complete reacted and the reaction of monoaddition product was completed. Then 0.35g (3mmol) of hydroxyethyl acrylate was added, and continued to stir for reaction under heat preservation. A sample was taken for testing every 4 hours of reaction time. The reaction was stopped until the hydroxyethyl acrylate content no longer decreases. The temperature was lowered to 30°C, 6.4g sodium carbonate aqueous solution with a mass concentration of 5% (3.0mmol sodium carbonate) was added, and then washed until neutral. The solvent was distilled off under reduced pressure to obtain 3.06g of the product of formula XV.

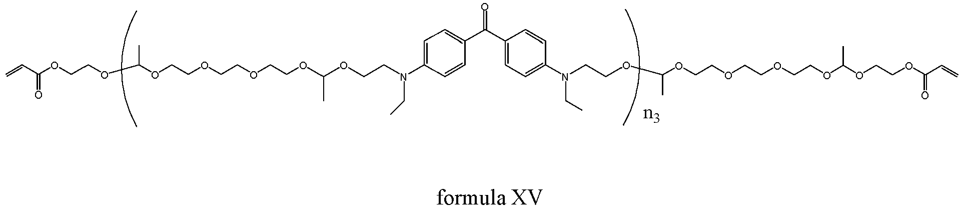

**Table 12 Analysis results of the product of Example 12 by liquid chromatography-mass spectrometry**

| Number | Retention time (min) | Content (%) | Molecular mass | Value of n₃ in formula XV |
|---|---|---|---|---|
| 1 | 7.45 | 18.67 | 993.20 | 1 |
| 2 | 11.83 | 19.63 | 1551.91 | 2 |
| 3 | 15.52 | 20.13 | 2110.63 | 3 |
| 4 | 1677 | 15.53 | 2669.35 | 4 |
| 5 | 18.91 | 8.80 | 3228.07 | 5 |
| 6 | 21.16 | 6.61 | 3786.79 | 6 |
| 7 | 24.42 | 4.38 | 4345.51 | 7 |
| 8 | 27.88 | 3.11 | 4904.23 | 8 |
| 9 | 29.33 | 1.99 | 5462.95 | 9 |
| 10 | 33.55 | 1.14 | 6021.67 | 10 |

### Example 13

To a 100mL three-necked flask with mechanical stirrer, 1.78g (5mmol) of HEMK, 1.42g (10mmol) of 1,4-butanediol divinyl ether, 0.022g (0.2mmol) of hydroquinone, 0.086g (0.5mmol) of p-aminobenzenesulfonic acid and 12.0g of toluene were added in sequence. After sufficient nitrogen replacement under room temperature with stirring, the mixture was heated and stirred for reaction under the nitrogen balloon sealing. The reaction temperature was 55°C and the reaction time was 48 hours. A sample was taken for HPLC detection and the reaction was stopped until HEMK was complete reacted and the reaction of monoaddition product was completed. Then 1.39g (12mmol) of hydroxyethyl acrylate was added, and continued to stir for reaction under heat preservation. A sample was taken for testing every 4 hours of reaction time. The reaction was stopped until the hydroxyethyl acrylate content no longer decreases. The temperature was lowered to 30°C, 6.4g sodium carbonate aqueous solution with a mass concentration of 5% (3.0mmol sodium carbonate) was added, and then washed until neutral. The solvent was distilled off under reduced pressure to obtain 4.09g of the product of formula XVI.

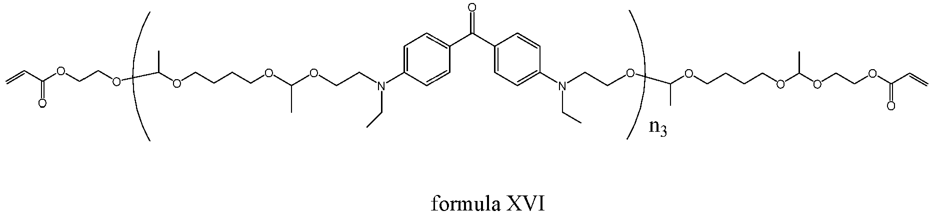

**Table 13 Analysis results of the product of Example 13 by liquid chromatography-mass spectrometry**

| Number | Retention time (min) | Content (%) | Molecular mass | Value of n₃ in formula XVI |
|---|---|---|---|---|
| 1 | 7.29 | 13.60 | 873.09 | 1 |
| 2 | 11.55 | 21.38 | 1371.76 | 2 |
| 3 | 16.04 | 17.89 | 1870.43 | 3 |
| 4 | 17.36 | 9.96 | 2369.10 | 4 |
| 5 | 20.64 | 15.08 | 2867.77 | 5 |
| 6 | 22.42 | 6.90 | 3366.44 | 6 |
| 7 | 23.34 | 5.46 | 3865.11 | 7 |
| 8 | 26.87 | 4.98 | 4363.78 | 8 |
| 9 | 28.91 | 3.22 | 4862.45 | 9 |
| 10 | 32.03 | 1.51 | 5361.12 | 10 |

### Example 14

This Example provided a photocurable composition, comprising the following components: 4.57g of Photomer 4072, 4.57g of Photomer 3316, 0.36g of the product of Formula IX of Example 1, and 0.5g of Omnipol TX.

The method for preparing the above-mentioned photocurable composition comprised the following steps: the above-mentioned components were stirred and dissolved at 60°C, and then cooled to room temperature to prepare a photocurable composition.

### Example 15

This Example provided a photocurable composition, comprising the following components: 4.57g of Photomer 4072, 4.57g of Photomer 3316, 0.36g of the product of Formula XI of Example 4, and 0.5g of Omnipol TX.

The method for preparing the above-mentioned photocurable composition comprised the following steps: the above-mentioned components were stirred and dissolved at 60°C, and then cooled to room temperature to prepare a photocurable composition.

### Example 16

This Example provided a photocurable composition, comprising the following components: 4.5g of Photomer 4072, 4.5g of Photomer 3316, 0.5g of the product of Formula XIII of Example 7, and 0.5g of Omnipol TX.

The method for preparing the above-mentioned photocurable composition comprised the following steps: the above-mentioned components were stirred and dissolved at 60°C, and then cooled to room temperature to prepare a photocurable composition.

### Example 17

This Example provided a photocurable composition, comprising the following components: 4.5g of Photomer 4072, 4.5g of Photomer 3316, 0.5g of the product of Formula XIV of Example 9, and 0.5g of Omnipol TX.

The method for preparing the above-mentioned photocurable composition comprised the following steps: the above-mentioned components were stirred and dissolved at 60°C, and then cooled to room temperature to prepare a photocurable composition.

### Example 18

This Example provided a photocurable composition, comprising the following components: 4.5g of Photomer 4072, 4.5g of Photomer 3316, 0.5g of the product of Formula XV of Example 11, and 0.5g of Omnipol TX.

The method for preparing the above-mentioned photocurable composition comprised the following steps: the above-mentioned components were stirred and dissolved at 60°C, and then cooled to room temperature to prepare a photocurable composition.

### Comparative Example 1

This comparative example provided a photocurable composition, comprising the following components: 4.65g of Photomer 4072, 4.65g of Photomer 3316, 0.2g of the Omnirad EMK, and 0.5g of Omnipol TX.

The method for preparing the above-mentioned photocurable composition comprised the following steps: the above-mentioned components were stirred and dissolved at 60°C, and then cooled to room temperature to prepare a photocurable composition.

### Test example

The hardness and curing mobility properties of the photocurable compositions prepared of Examples 14-18 and Comparative Example 1 were tested respectively:
Pendulum hardness test: a 25µm wire rod was used to cure the above-mentioned photocurable compositions on a coated glass plate (under a 395nm LED light) at a speed of 10m/min, and the pendulum hardness after curing was tested.

Mobility test: a 25µm wire rod was used to cure the above-mentioned photocurable composition on a paper with a coating length and width of 5×20cm under a 395nm LED lamp at a speed of 10m/min. The 100cm² of cured paper was put into 100g of acetic acid aqueous solution with a mass content of 3%, then it was placed at 40°C for 10 days. Then HPLC was used to detect the photoinitiator component (the photoinitiator component refers to the product component of the formula IX of Example 1, the product component of Formula XI of Example 4, the product component of Formula XIII of Example 7, the product component of Formula XIV of Example 9, the product component of Formula XV of Example 11 or the component of Omnirad EMK) that migrates into the acetic acid aqueous solution. The EU model was used for calculation of the results, assuming that 1kg of food is packaged in a 600cm² printing area, so the results can be converted into µg/kg, that is, µg of analyte per kg of food (the analyte refers to the product of formula IX of Example 1, the product of formula XI of Example 4, the product of formula XIII of Example 7, the product of formula XIV of Example 9, the product of formula XV of Example 11 or Omnirad EMK). The experimental results of hardness and mobility analysis were shown in Table 14.

**Table 14**

| | Example 14 | Example 15 | Example 16 | Example 17 | Example 18 | Comparative Example 1 |
|---|---|---|---|---|---|---|
| Pendulum hardness | 0.76 | 0.75 | 0.75 | 0.79 | 0.80 | 0.77 |
| Mobility (µg/kg) | 25 | 23 | 27 | 11 | 6 | 2180 |

It can be seen from the test data that using the bis(dialkylamino)benzophenone compound with an acrylate alkoxy side chain provided by the present application as a co-initiator of the photocurable composition, compared with the commonly used EMK as a co-initiator for photocurable compositions on the market, the hardness of the compounds provided in the present application after curing is similar to that of the control, indicating that they have a similar curing rate. However, because the compound provided by the application has a larger molecular weight and contains polymerizable double bonds, the mobility is significantly reduced. Therefore, the compounds provided in the present application are more suitable for use in food and drug packaging, children's toys and other applications that have strict requirements on substance mobility.

Obviously, the above examples are merely examples made for clear description, rather limiting the implementations. For those of ordinary skill in the art, other different forms of variations or modifications can also be made on the basis of the above-mentioned description. All embodiments are not necessary to be and cannot be exhaustively listed herein.

## Claims

1. A benzophenone derivative, wherein the benzophenone derivative has a structure represented by formula (1): 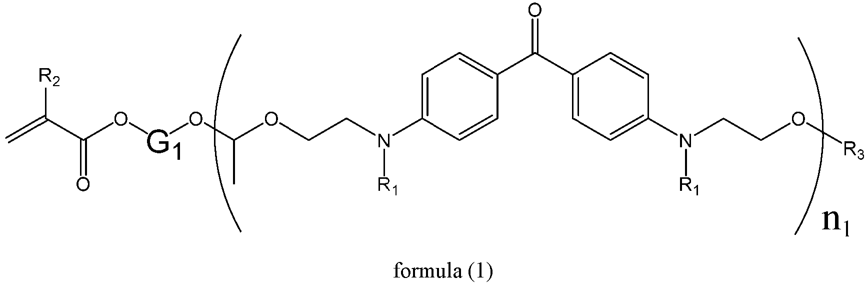 wherein:
n₁ is an integer from 1 to 10, R₁ is an optionally substituted C1-C8 alkyl, R₂ is H or -CH₃,
R₃ is H or 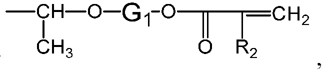 G₁ is an optionally substituted C1-C12 alkylene, 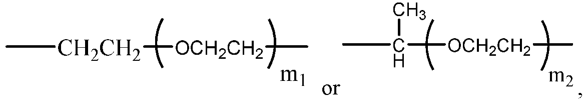 m₁ is an integer from 1 to 11, and m₂ is an integer from 1 to 12.

2. The benzophenone derivative of claim 1, wherein n₁ is an integer from 1 to 4; preferably, R₁ is a C1-C4 alkyl, further preferably -CH₂CH₃; preferably, R₂ is H;
preferably, R₃ is 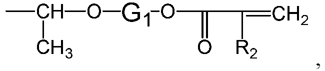
R₂ and G₁ have the same definitions as those in formula (1) of claim 1; and
preferably, G₁ is -CH₂CH₂-, -CH₂CH₂CH₂CH₂-, or -CH₂CH₂OCH₂CH₂-.

3. A benzophenone derivative, wherein the benzophenone derivative has a structure represented by formula (2): 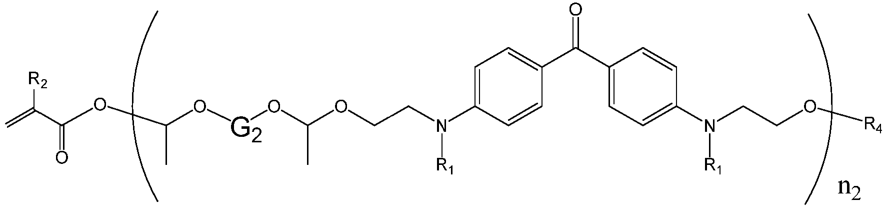 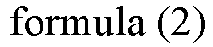 wherein:
n₂ is an integer from 1 to 10, R₁ is an optionally substituted C1-C8 alkyl, R₂ is H or -CH₃,
R₄ is H or 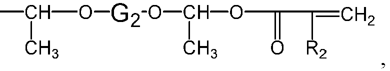
G₂ is an optionally substituted C1-C12 alkylene or 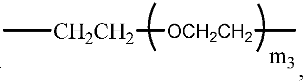 and m₃ is an integer from 1 to 11.

4. The benzophenone derivative of claim 3, wherein n₂ is an integer from 1 to 6;
preferably, R₁ is preferably a C1-C4 alkyl, further preferably -CH₂CH₃; preferably, R₂ is H; preferably, R₄ is 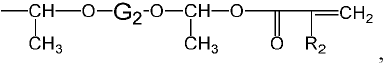 the R₂ and G₂ have the same definitions as those in formula (2) of claim 3.

5. A benzophenone derivative, wherein the benzophenone derivative has a structure represented by formula (3): 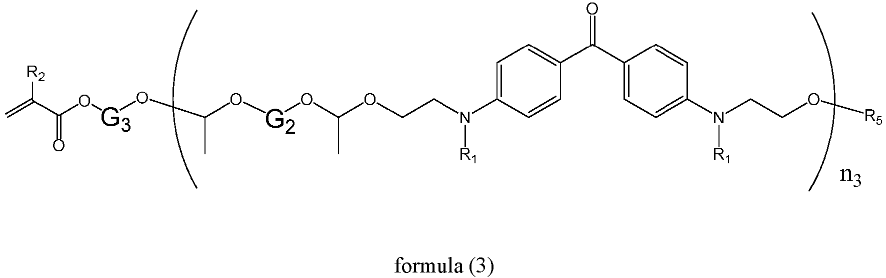 wherein:
n₃ is an integer from 1 to 10, R₁ is an optionally substituted C1-C8 alkyl, R₂ is H or -CH₃, R₅ is H or 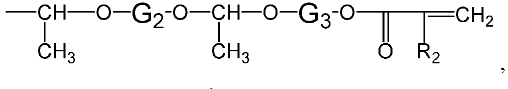 G₂ is an optionally substituted C1-C12 alkylene or 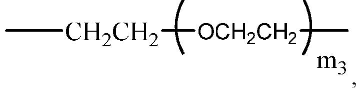 m₃ is an integer from 1 to 11; G₃ is an optionally substituted C1-C12 alkylene, 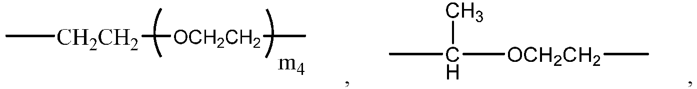 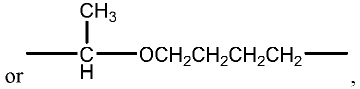 and m₄ is an integer from 1 to 11.

6. The benzophenone derivative of claim 5, wherein n₃ is an integer from 1 to 6; preferably, R₁ is a C1-C4 alkyl, further preferably -CH₂CH₃; preferably, R₂ is H;
preferably, R₅ is 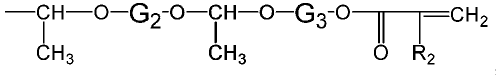 , the R₂, G₂ and G₃ have the same definitions as those in formula (3) of claim 5; and
preferably, G₂ and G₃ are each independently -CH₂CH₂-, -CH₂CH₂CH₂CH₂-, -CH₂CH₂OCH₂CH₂- or -CH₂CH₂OCH₂CH₂OCH₂CH₂-.

7. A method for preparing the benzophenone derivative of claim 1 or 2, comprising:
performing a reaction between the compound represented by formula (4) and the compound represented by formula (5) until at least one of the hydroxyl groups at both ends of the compound represented by formula (4) is fully added, and then ending the reaction to obtain a benzophenone derivative with the structure of formula (1); 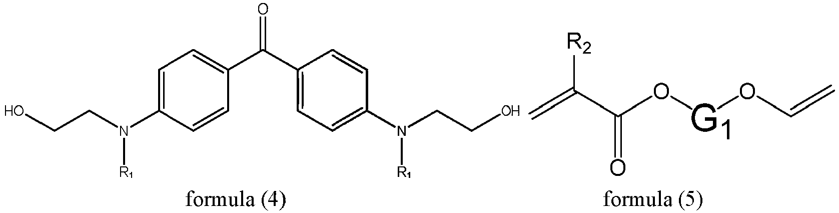
wherein a molar ratio of the compound represented by formula (4) to the compound represented by formula (5) is in a range from 1:1 to 1:2, and R₁, R₂ and G₁ have the same definitions as those in formula (1); preferably the reaction temperature is in a range from 40°C to 120°C and the reaction time is in a range from 10h to 100h.

8. A method for preparing the benzophenone derivative of claim 3 or 4, comprising:
performing a reaction between the compound represented by formula (6) and the compound represented by formula (7) until at least one of the hydroxyl groups at both ends of the compound represented by formula (6) is fully added, adding the compound represented by formula (8) and continuing to react until the content of the compound represented by equation (8) no longer decreases, and stopping the reaction; 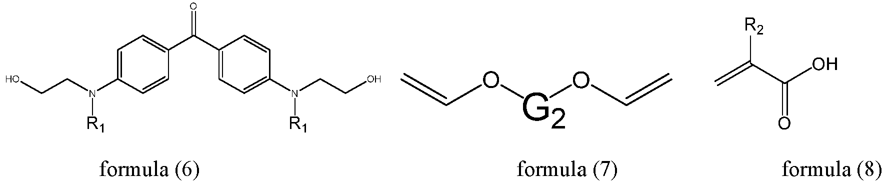
wherein a molar ratio of the compound represented by formula (6) to the compound represented by formula (7) is in a range from 1:1 to 1:2, and a molar ratio of the compound represented by formula (6) to the compound represented by formula (8) is in a range from 1:0.5 to 1:3, and R₁, R₂ and G₂ have the same definitions as those in formula (2); preferably the reaction temperature is in a range from 40°C to 120°C and the reaction time is in a range from 10h to 100h.

9. A method for preparing benzophenone derivatives of claim 5 or 6, comprising:
performing a reaction the compound represented by formula (9) with the compound represented by formula (10) until at least one of the hydroxyl groups at both ends of the compound represented by formula (9) is fully added, then adding the compound represented by formula (11), and continuing to react until all double bonds in the vinyl of the compound represented by formula (11) are added; 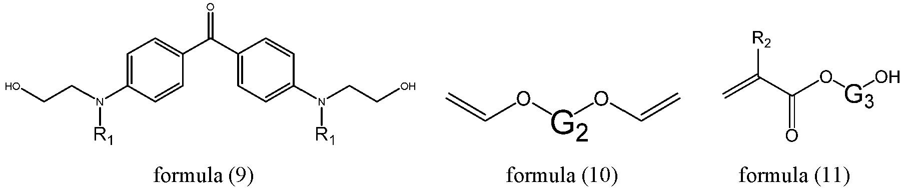
wherein a molar ratio of the compound represented by formula (9) and the compound represented by formula (10) is 1:1 to 1:2, and the molar ratio of the compound represented by formula (9) and the compound represented by formula (11) is 1:1 to 1:3, and R₁, R₂, G₂ and G₃ have the same definitions as those in formula (3) ; preferably the reaction temperature is in a range from 40°C to 120°C and the reaction time is in a range from 10h to 100h.

10. A photoinitiator composition, wherein the photoinitiator composition comprises a photoinitiator for free radical polymerization and the benzophenone derivative of any one of claims 1 to 6.

11. A photocurable composition, wherein the photocurable composition comprises a photoinitiator component and a radically polymerizable ethylenically unsaturated compound, and the photoinitiator component comprises the photoinitiator composition of claim 10.

12. The photocurable composition of claim 11, wherein the photocurable composition comprises:
(a) the benzophenone derivative of any one of claims 1 to 6;
(b) a photoinitiator for free radical polymerization; and
(c) a free-radically polymerizable ethylenically unsaturated compound; preferably the component (a) is added in an amount ranging from from 0.1% to 20% of the total weight of the photocurable composition; or
the component (b) is added in an amount ranging from 0.1% to 10% of the total weight of the photocurable composition; orwherein the component (b) is selected from the group consisting of benzophenones, thioxanthone compounds, α-hydroxyketone compounds, α-aminoketones compounds, acyl phosphine oxide compounds, oxime lipid compounds, and any combination thereof; preferably, at least one of benzophenone, 2-isopropylthiaxanthone, 2-dimethylamino-2-(4-methyl benzyl)-1-(4-morpholinylphenyl)-1-butanone, 2,4,6-trimethylbenzoyl-diphenylphosphine oxide, photoinitiator Omnipol TX, photoinitiator Omnipol 910 and photoinitiator Omnipol TP; or
the component (c) is at least one selected from the group consisting of epoxy acrylate resin, polyurethane acrylate resin, polyester acrylate resin, polyether acrylate resin, and acrylate resin, polyacrylate, epoxy methacrylate resin, polyurethane methacrylate resin, polyester methacrylate resin, polyether methacrylate resin, acrylated polymethacrylate, allyl ether compound, acrylate monomer and methacrylate monomer.

13. Use of the photocurable composition of claim 11 or 12 in food packaging printing, pharmaceutical packaging printing, furniture coating, book printing and advertising printing.

14. A photocurable product, wherein the photocurable product is formed by photocuring a photocurable composition, wherein the photocurable composition is the photocurable composition of claim 11 or 12.

15. A method for curing a photocurable composition, comprising
coating the photocurable composition of claim 11 or 12 on a substrate; and curing the photocurable composition by using a light source with an emission band in the UV-visible light region; preferably the substrate is selected from the group consisting of wood, paper, plastic, coating and metal; or the coating method is selected from the group consisting of offset printing, gravure printing, flexographic printing, inkjet printing and 3D printing; or
after coating on the substrate, the photocurable composition is cured by irradiating with UV-visible light having a wavelength ranging from 200 nm to 425 nm; preferably, the photocurable composition is cured by irradiating with UV-visible light having a wavelength ranging from 365 nm to 405 nm.

## Patentansprüche

1. Benzophenonderivat, wobei das Benzophenonderivat eine Struktur dargestellt durch Formel (1) aufweist: 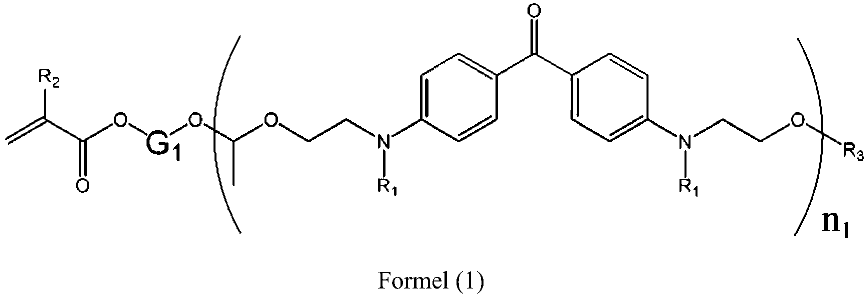 wobei:
n₁ eine ganze Zahl von 1 bis 10 ist, R₁ ein optional substituiertes C1-C8-Alkyl ist, R₂ H oder -CH₃ ist,
R₃ H oder 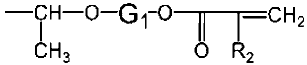 ist, G₁ ein optional substituiertes C1-C12-Alkylen, 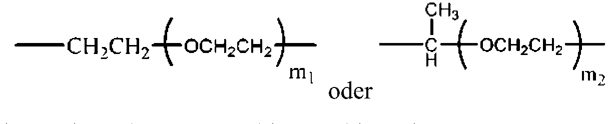 ist, m₁ eine ganze Zahl von 1 bis 11 ist, und m₂ eine ganze Zahl von 1 bis 12 ist.

2. Benzophenonderivat nach Anspruch 1, wobei n₁ eine ganze Zahl von 1 bis 4 ist;
bevorzugt R₁ ein C1-C4-Alkyl, ferner bevorzugt -CH₂CH₃ ist; bevorzugt R₂ H ist;
bevorzugt R₃ 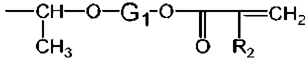 ist, R₂ und G₁ die gleichen Definitionen wie diejenigen in Formel (1) von Anspruch 1 aufweisen; und
bevorzugt G₁ -CH₂CH₂-, -CH₂CH₂CH₂CH₂- oder -CH₂CH₂OCH₂CH₂- ist.

3. Benzophenonderivat, wobei das Benzophenonderivat eine Struktur dargestellt durch Formel (2) aufweist: 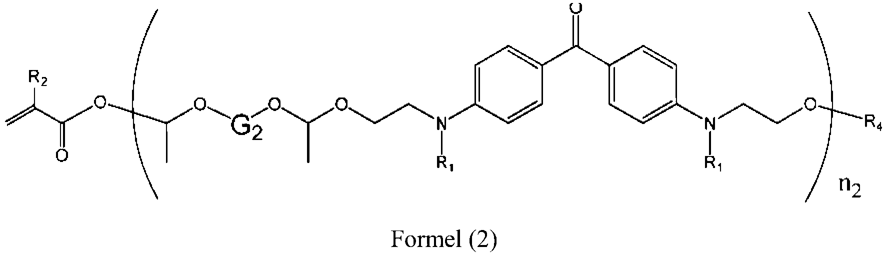 wobei:
n₂ eine ganze Zahl von 1 bis 10 ist, R₁ ein optional substituiertes C1-C8-Alkyl ist, R₂ H oder -CH₃ ist,
R₄ H oder 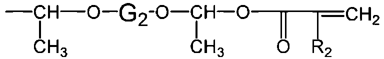 ist,
G₂ ein optional substituiertes C1-C12-Alkylen oder 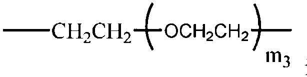 ist und m₃ eine ganze Zahl von 1 bis 11 ist.

4. Benzophenonderivat nach Anspruch 3, wobei n₂ eine ganze Zahl von 1 bis 6 ist;
bevorzugt R₁ bevorzugt ein C1-C4-Alkyl, ferner bevorzugt -CH₂CH₃ ist; bevorzugt R₂ H ist;
bevorzugt R₄ 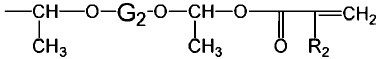 ist, das R₂ und G₂ die gleichen Definitionen wie diejenigen in Formel (2) von Anspruch 3 aufweisen.

5. Benzophenonderivat, wobei das Benzophenonderivat eine Struktur dargestellt durch Formel (3) aufweist: 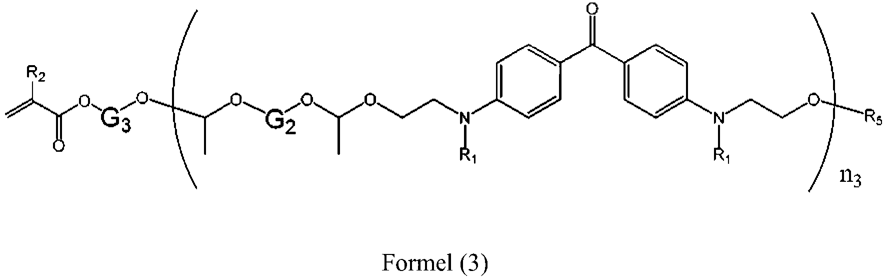 wobei:
n₃ eine ganze Zahl von 1 bis 10 ist, R₁ ein optional substituiertes C1-C8-Alkyl ist, R₂ H oder -CH₃ ist, R₅ H oder 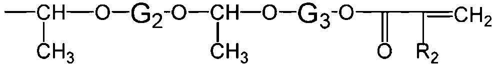 ist, G₂ ein optional substituiertes C1-C12-Alkylen oder 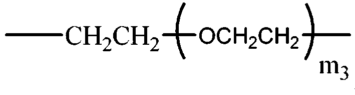 ist, m₃ eine ganze Zahl von 1 bis 11 ist;
G₃ ein optional substituiertes C1-C12-Alkylen, 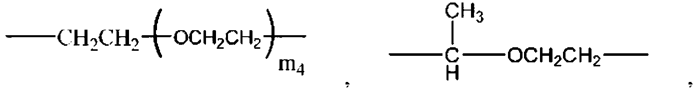
oder 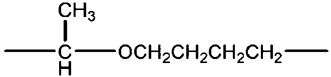 ist und m₄ eine ganze Zahl von 1 bis 11 ist.

6. Benzophenonderivat nach Anspruch 5, wobei n₃ eine ganze Zahl von 1 bis 6 ist;
bevorzugt R₁ ein C1-C4-Alkyl, ferner bevorzugt -CH₂CH₃ ist; bevorzugt R₂ H ist;
bevorzugt R₅ 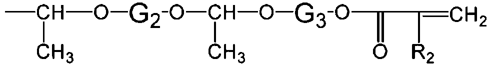 ist, das R₂, G₂ und G₃ die gleichen Definitionen wie diejenigen in Formel (3) von Anspruch 5 aufweisen; und
bevorzugt G₂ und G₃ jeweils unabhängig -CH₂CH₂-, -CH₂CH₂CH₂CH₂-, -CH₂CH₂OCH₂CH₂- oder -CH₂CH₂OCH₂CH₂OCH₂CH₂- sind.

7. Verfahren zur Herstellung des Benzophenonderivats nach Anspruch 1 oder 2, umfassend:
Durchführen einer Reaktion zwischen der Verbindung dargestellt durch Formel (4) und der Verbindung dargestellt durch Formel (5), bis zumindest eine der Hydroxylgruppen an beiden Enden der Verbindung dargestellt durch Formel (4) vollständig hinzugefügt ist, und dann Beenden der Reaktion, um ein Benzophenonderivat mit der Struktur der Formel (1) zu erhalten; 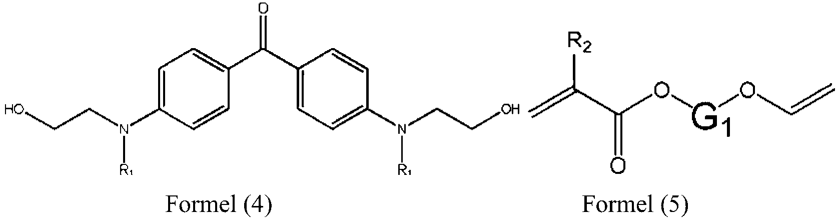
wobei ein Molverhältnis der Verbindung dargestellt durch Formel (4) zu der Verbindung dargestellt durch Formel (5) in einer Spanne von 1:1 bis 1:2 ist und R₁, R₂ und G₁ die gleichen Definitionen wie diejenigen in Formel (1) aufweisen; bevorzugt die Reaktionstemperatur in einer Spanne von 40 °C bis 120 °C ist und die Reaktionszeit in einer Spanne von 10h bis 100h ist.

8. Verfahren zur Herstellung des Benzophenonderivats nach Anspruch 3 oder 4, umfassend:
Durchführen einer Reaktion zwischen der Verbindung dargestellt durch Formel (6) und der Verbindung dargestellt durch Formel (7), bis zumindest eine der Hydroxylgruppen an beiden Enden der Verbindung dargestellt durch Formel (6) vollständig hinzugefügt ist, Hinzufügen der Verbindung dargestellt durch Formel (8) und Fortsetzen, zu reagieren, bis der Gehalt der Verbindung dargestellt durch Gleichung (8) nicht länger abnimmt, und Stoppen der Reaktion; 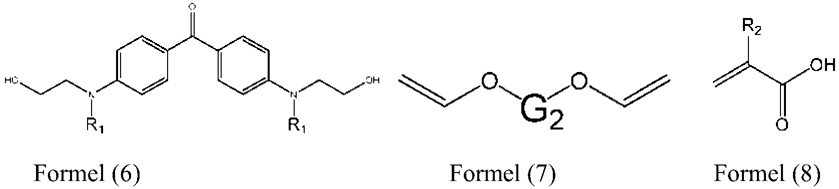
wobei ein Molverhältnis der Verbindung dargestellt durch Formel (6) zu der Verbindung dargestellt durch Formel (7) in einer Spanne von 1:1 bis 1:2 ist und ein Molverhältnis der Verbindung dargestellt durch Formel (6) zu der Verbindung dargestellt durch Formel (8) in einer Spanne von 1:0,5 bis 1:3 ist und R₁, R₂ und G₂ die gleichen Definitionen wie diejenigen in Formel (2) aufweisen; bevorzugt die Reaktionstemperatur in einer Spanne von 40 °C bis 120 °C ist und die Reaktionszeit in einer Spanne von 10h bis 100h ist.

9. Verfahren zur Herstellung von Benzophenonderivaten nach Anspruch 5 oder 6, umfassend:
Durchführen einer Reaktion der Verbindung dargestellt durch Formel (9) mit der Verbindung dargestellt durch Formel (10), bis zumindest eine der Hydroxylgruppen an beiden Enden der Verbindung dargestellt durch Formel (9) vollständig hinzugefügt ist, dann Hinzufügen der Verbindung dargestellt durch Formel (11) und Fortsetzen, zu reagieren, bis alle Doppelbindungen in dem Vinyl der Verbindung dargestellt durch Formel (11) hinzugefügt sind; 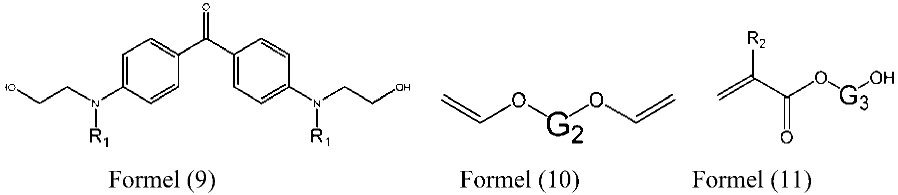
wobei ein Molverhältnis der Verbindung dargestellt durch Formel (9) und der Verbindung dargestellt durch Formel (10) 1:1 bis 1:2 ist und das Molverhältnis der Verbindung dargestellt durch Formel (9) und der Verbindung dargestellt durch Formel (11) 1:1 bis 1:3 ist und R₁, R₂, G₂ und G₃ die gleichen Definitionen wie diejenigen in Formel (3) aufweisen; bevorzugt die Reaktionstemperatur in einer Spanne von 40 °C bis 120 °C ist und die Reaktionszeit in einer Spanne von 10h bis 100h ist.

10. Photoinitiatorzusammensetzung, wobei die Photoinitiatorzusammensetzung einen Photoinitiator für freie radikalische Polymerisation und das Benzophenonderivat nach einem der Ansprüche 1 bis 6 umfasst.

11. Photohärtbare Zusammensetzung, wobei die photohärtbare Zusammensetzung eine Photoinitiatorkomponente und eine radikalisch polymerisierbare ethylenisch ungesättigte Verbindung umfasst und die Photoinitiatorkomponente die Photoinitiatorzusammensetzung nach Anspruch 10 umfasst.

12. Photohärtbare Zusammensetzung nach Anspruch 11, wobei die photohärtbare Zusammensetzung Folgendes umfasst:
(a) das Benzophenonderivat nach einem der Ansprüche 1 bis 6;
(b) einen Photoinitiator für freie radikalische Polymerisation; und
(c) eine frei-radikalisch polymerisierbare ethylenisch ungesättigte Verbindung; wobei bevorzugt die Komponente (a) in einer Menge hinzugefügt wird, die von 0,1 % bis 20 % des Gesamtgewichts der photohärtbaren Zusammensetzung reicht; oder
die Komponente (b) in einer Menge hinzugefügt wird, die von 0,1 % bis 10 % des Gesamtgewichts der photohärtbaren Zusammensetzung reicht; oder wobei die Komponente (b) ausgewählt ist aus der Gruppe bestehend aus Benzophenonen, Thioxanthonverbindungen, α-Hydroxyketonverbindungen, α-Aminoketonverbindungen, Acylphosphinoxidverbindungen, Oximlipidverbindungen und einer beliebigen Kombination davon; bevorzugt zumindest einem von Benzophenon, 2-Isopropylthiaxanthon, 2-Dimethylamino-2-(4-methylbenzyl)-1-(4-morpholinylphenyl)-1-butanon, 2,4,6-Trimethylbenzoyl-diphenylphosphinoxid, Photoinitiator Omnipol TX, Photoinitiator Omnipol 910 und Photoinitiator Omnipol TP; oder
die Komponente (c) zumindest eine ausgewählt aus der Gruppe bestehend aus Epoxidacrylatharz, Polyurethanacrylatharz, Polyesteracrylatharz, Polyetheracrylatharz und Acrylatharz, Polyacrylat, Epoxidmethacrylatharz, Polyurethanmethacrylatharz, Polyestermethacrylatharz, Polyethermethacrylatharz, acryliertem Polymethacrylat, Allyletherverbindung, Acrylatmonomer und Methacrylatmonomer ist.

13. Verwendung der photohärtbaren Zusammensetzung nach Anspruch 11 oder 12 in Lebensmittelverpackungsdruck, pharmazeutischem Verpackungsdruck, Möbelbeschichtung, Buchdruck und Werbedruck.

14. Photohärtbares Produkt, wobei das photohärtbare Produkt durch Photohärten einer photohärtbaren Zusammensetzung gebildet ist, wobei die photohärtbare Zusammensetzung die photohärtbare Zusammensetzung nach Anspruch 11 oder 12 ist.

15. Verfahren zur Härtung einer photohärtbaren Zusammensetzung, umfassend Beschichten der photohärtbaren Zusammensetzung nach Anspruch 11 oder 12 auf einem Substrat; und Härten der photohärtbaren Zusammensetzung durch Verwenden einer Lichtquelle mit einem Emissionsband in der UV-sichtbaren Lichtregion; wobei bevorzugt das Substrat ausgewählt ist aus der Gruppe bestehend aus Holz, Papier, Kunststoff, Beschichtung und Metall; oder das Beschichtungsverfahren ausgewählt ist aus der Gruppe bestehend aus Offsetdruck, Tiefdruck, Flexodruck, Tintenstrahldruck und 3D-Druck; oder
nach dem Beschichten auf dem Substrat die photohärtbare Zusammensetzung durch Bestrahlen mit UV-sichtbarem Licht mit einer Wellenlänge gehärtet wird, die von 200 nm bis 425 nm reicht; bevorzugt die photohärtbare Zusammensetzung durch Bestrahlen mit UV-sichtbarem Licht mit einer Wellenlänge gehärtet wird, die von 365 nm bis 405 nm reicht.

## Revendications

1. Dérivé de benzophénone, ledit dérivé de benzophénone présentant une structure représentée par la formule (1) : 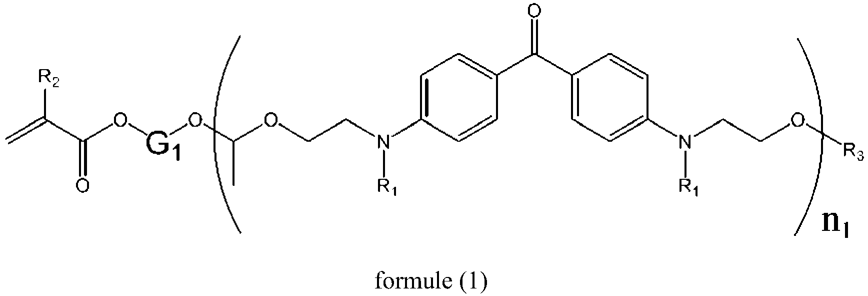 dans lequel :
n₁ est un entier de 1 à 10, R₁ est un alkyle en C1-C8 éventuellement substitué, R₂ est H ou -CH₃, R₃ est H ou 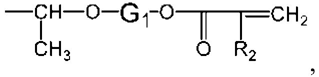 G₁ est un alkylène en C1-C12 éventuellement substitué, 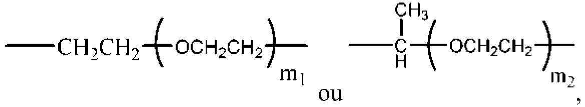 m₁ est un entier de 1 à 11, et m₂ est un entier de 1 à 12.

2. Dérivé de benzophénone de la revendication 1, dans lequel n₁ est un entier de 1 à 4 ;
de préférence, R₁ est un alkyle en C1-C4, de préférence encore -CH₂CH₃ ; de préférence, R₂ est H ;
de préférence, R₃ est 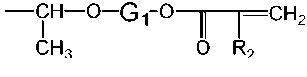 , R₂ et G₁ ont les mêmes définitions que celles de la formule (1) de la revendication 1 ; et
de préférence, G₁ est -CH₂CH₂-, -CH₂CH₂CH₂CH₂- ou -CH₂CH₂OCH₂CH₂-,

3. Dérivé de benzophénone, ledit dérivé de benzophénone présentant une structure représentée par la formule (2) : 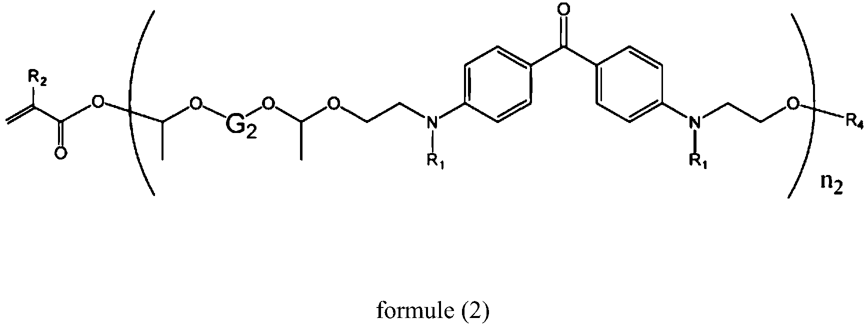 dans lequel :
n₂ est un entier de 1 à 10, R₁ est un alkyle en C1-C8 éventuellement substitué, R₂ est H ou -CH₃, R₄ est H ou 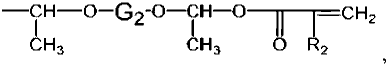
G₂ est un alkylène en C1-C12 éventuellement substitué ou 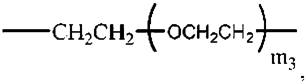 et m₃ est un entier de 1 à 11.

4. Dérivé de benzophénone de la revendication 3, dans lequel n₂ est un entier de 1 à 6 ;
de préférence, R₁ est de préférence un alkyle en C1-C4, de préférence encore -CH₂CH₃ ; de préférence, R₂ est H ;
de préférence, R₄ est 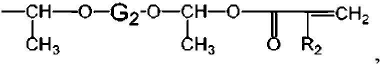 R₂ et G₂ ont les mêmes définitions que celles de la formule (2) de la revendication 3.

5. Dérivé de benzophénone, ledit dérivé de benzophénone présentant une structure représentée par la formule (3) : 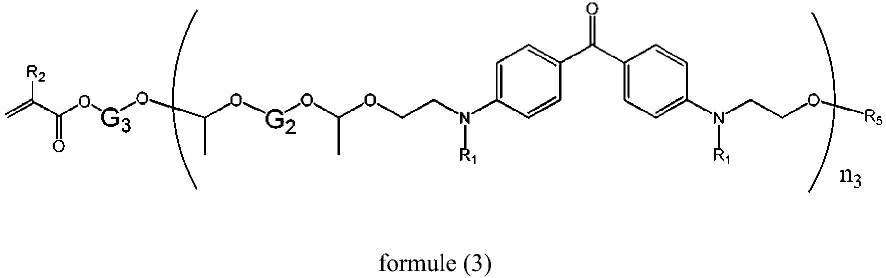 dans lequel :
n₃ est un entier de 1 à 10, R₁ est un alkyle en C1-C8 éventuellement substitué, R₂ est H ou -CH₃, R₅ est H ou 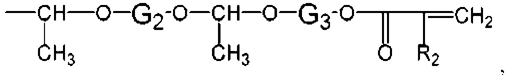 G₂ est un alkylène en C1-C12 éventuellement
substitué ou 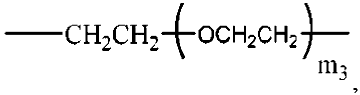 m₃ est un entier de 1 à 11 ; G₃ est un alkylène en C1-C12 éventuellement substitué, 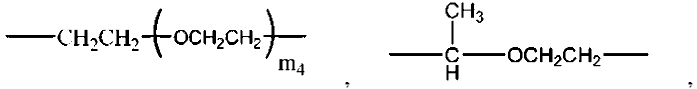
ou 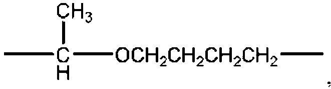 et m₄ est un entier de 1 à 11.

6. Dérivé de benzophénone de la revendication 5, dans lequel n₃ est un entier de 1 à 6 ;
de préférence, R₁ est un alkyle en C1-C4, de préférence encore -CH₂CH₃ ; de préférence, R₂ est H ;
de préférence, R₅ est 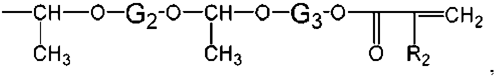
R₂, G₂ et G₃ ont les mêmes définitions que celles de la formule (3) de la revendication 5 ; et
de préférence, G₂ et G₃ sont chacun indépendamment -CH₂CH₂-, -CH₂CH₂CH₂CH₂-, -CH₂CH₂OCH₂CH₂- ou -CH₂CH₂OCH₂CH₂OCH₂CH₂-.

7. Procédé permettant la préparation du dérivé de benzophénone de l'une des revendications 1 ou 2, comprenant :
la réalisation d'une réaction entre le composé représenté par la formule (4) et le composé représenté par la formule (5) jusqu'à ce qu'au moins l'un des groupes hydroxyle aux deux extrémités du composé représenté par la formule (4) soit entièrement ajouté, puis l'arrêt de la réaction pour obtenir un dérivé de benzophénone de la structure de formule (1) ; 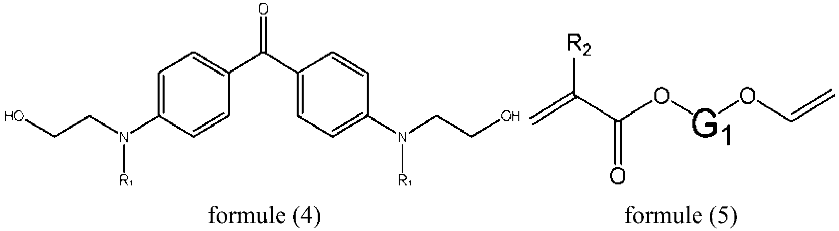
dans lequel un rapport molaire du composé représenté par la formule (4) au composé représenté par la formule (5) est compris dans une plage de 1:1 à 1:2, et R₁, R₂ et G₁ ont les mêmes définitions que celles de la formule (1) ; de préférence, la température de réaction est comprise dans une plage de 40 °C à 120 °C et le temps de réaction est compris dans une plage de 10 h à 100 h.

8. Procédé permettant la préparation du dérivé de benzophénone de l'une des revendications 3 ou 4, comprenant :
la réalisation d'une réaction entre le composé représenté par la formule (6) et le composé représenté par la formule (7) jusqu'à ce qu'au moins l'un des groupes hydroxyle aux deux extrémités du composé représenté par la formule (6) soit entièrement ajouté, l'ajout du composé représenté par la formule (8) et la poursuite de la réaction jusqu'à ce que la teneur en composé représenté par l'équation (8) ne diminue plus, et l'arrêt de la réaction ; 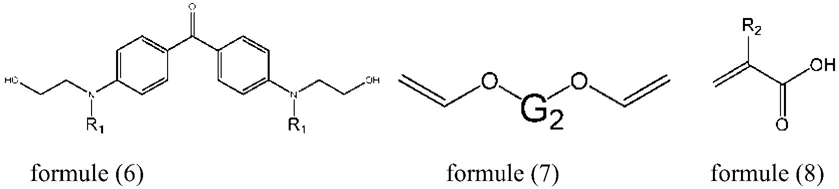
dans lequel un rapport molaire du composé représenté par la formule (6) au composé représenté par la formule (7) est compris dans une plage de 1:1 à 1:2, et un rapport molaire du composé représenté par la formule (6) au composé représenté par la formule (8) est compris dans une plage de 1:0,5 à 1:3, et R₁, R₂ et G₂ ont les mêmes définitions que celles de la formule (2) ; de préférence, la température de réaction est comprise dans une plage de 40 °C à 120 °C et le temps de réaction est compris dans une plage de 10 h à 100 h.

9. Procédé permettant la préparation de dérivés de benzophénone de l'une des revendications 5 ou 6, comprenant :
la réalisation d'une réaction du composé représenté par la formule (9) avec le composé représenté par la formule (10) jusqu'à ce qu'au moins l'un des groupes hydroxyle aux deux extrémités du composé représenté par la formule (9) soit entièrement ajouté, puis l'ajout du composé représenté par la formule (11), et la poursuite de la réaction jusqu'à ce que toutes les doubles liaisons dans le vinyle du composé représenté par la formule (11) soient ajoutées ; 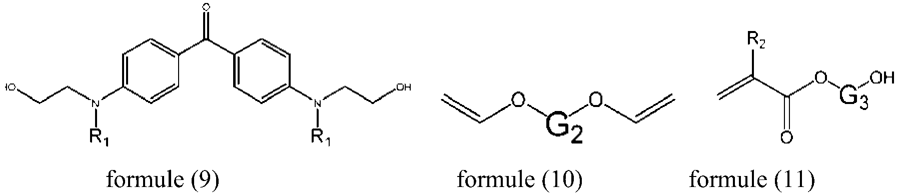
dans lequel un rapport molaire du composé représenté par la formule (9) au composé représenté par la formule (10) est de 1:1 à 1:2, et le rapport molaire du composé représenté par la formule (9) au composé représenté par la formule (11) est de 1:1 à 1:3, et R₁, R₂, G₂ et G₃ ont les mêmes définitions que celles de la formule (3) ; de préférence, la température de réaction est comprise dans une plage de 40 °C à 120 °C et le temps de réaction est compris dans une plage de 10 h à 100 h.

10. Composition de photoinitiateur, ladite composition de photoinitiateur comprenant un photoinitiateur pour la polymérisation radicalaire et le dérivé de benzophénone de l'une quelconque des revendications 1 à 6.

11. Composition photodurcissable, ladite composition photodurcissable comprenant un composant photoinitiateur et un composé éthyléniquement insaturé polymérisable par voie radicalaire, et le composant photoinitiateur comprend la composition de photoinitiateur de la revendication 10.

12. Composition photodurcissable de la revendication 11, ladite composition photodurcissable comprenant :
(a) le dérivé de benzophénone de l'une quelconque des revendications 1 à 6 ;
(b) un photoinitiateur pour la polymérisation radicalaire ; et
(c) un composé éthyléniquement insaturé polymérisable par voie radicalaire ; de préférence, le composant (a) est ajouté en une quantité allant de 0,1 % à 20 % du poids total de la composition photodurcissable ; ou
le composant (b) est ajouté en une quantité allant de 0,1 % à 10 % du poids total de la composition photodurcissable ; ou dans lequel le composant (b) est choisi dans le groupe constitué de benzophénones, de composés de thioxanthone, de composés d'α-hydroxycétone, de composés d'α-aminocétones, de composés d'oxyde d'acylphosphine, de composés lipidiques d'oxime et de toute combinaison de ceux-ci ; de préférence, au moins l'un de la benzophénone, de la 2-isopropylthiaxanthone, de la 2-diméthylamino-2-(4-méthylbenzyl)-1-(4-morpholinylphényl)-1-butanone, de l'oxyde de 2,4,6-triméthylbenzoyl-diphénylphosphine, du photoinitiateur Omnipol TX, du photoinitiateur Omnipol 910 et du photoinitiateur Omnipol TP ; ou
le composant (c) est au moins un composé choisi dans le groupe constitué par une résine époxy acrylate, une résine polyuréthane acrylate, une résine polyester acrylate, une résine polyéther acrylate et une résine acrylate, un polyacrylate, une résine époxy méthacrylate, une résine polyuréthane méthacrylate, une résine polyester méthacrylate, une résine polyéther méthacrylate, un polyméthacrylate acrylé, un composé d'éther allylique, un monomère acrylate et un monomère méthacrylate.

13. Utilisation de la composition photodurcissable de l'une des revendications 11 ou 12 dans l'impression d'emballages alimentaires, l'impression d'emballages pharmaceutiques, le revêtement de meubles, l'impression de livres et l'impression publicitaire.

14. Produit photodurcissable, ledit produit photodurcissable étant formé par photodurcissement d'une composition photodurcissable, ladite composition photodurcissable étant la composition photodurcissable de l'une des revendications 11 ou 12.

15. Procédé permettant le durcissement d'une composition photodurcissable, comprenant
le dépôt de la composition photodurcissable de l'une des revendications 11 ou 12 sur un substrat ; et
le durcissement de la composition photodurcissable en utilisant une source de lumière avec une bande d'émission dans la région de la lumière UV-visible ; de préférence, le substrat est choisi dans le groupe constitué de bois, de papier, de matière plastique, de revêtement et de métal ; ou le procédé de revêtement est choisi dans le groupe constitué de l'impression offset, de l'héliogravure, de l'impression flexographique, de l'impression à jet d'encre et de l'impression 3D ; ou
après dépôt sur le substrat, la composition photodurcissable est durcie par irradiation avec une lumière UV-visible possédant une longueur d'onde allant de 200 nm à 425 nm ; de préférence, la composition photodurcissable est durcie par irradiation avec une lumière UV-visible possédant une longueur d'onde allant de 365 nm à 405 nm.
